# EUROPEAN PATENT APPLICATION

(11) **EP 4 813 988 A1**
(43) Date of publication of application: **30.09.2026**
(21) Application number: 25829704.3
(22) Date of filing: 10.06.2025
(51) Int. Cl.: C07D 471/04, C07D 243/38, C07D 401/12, C07D 401/14, A61K 31/5513, A61P 27/10

(54) **AI-DESIGNED BENZODIAZEPINE DERIVATIVE AND USE THEREOF**

(30) Priority: 18.06.2024 CN 202410793543
(71) Applicant: Shenzhen Newrosetta Biosciences Co., Ltd, Shenzhen, Guangdong 518000 (CN)
(72) Inventor: YI, Jihui, Shenzhen, Guangdong 518000 (CN); YU, Jindi, Shenzhen, Guangdong 518000 (CN)
(74) Representative: Huang, Liwei
(86) International application number: PCT/CN2025/100156
(87) International publication number: WO 2025/261223

(57) **Abstract**

Disclosed in the present invention are an Ai-designed benzodiazepine derivative and a use thereof. The benzodiazepine derivative has a structure of Formula I, 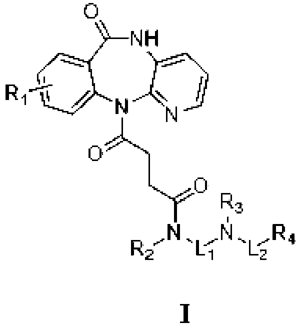 wherein the definition of each group is as described in the description. The benzodiazepine derivative provided by the present invention, as an active component of a pharmaceutical composition, can significantly improve selectivity for an M2 receptor and significantly reduce the inhibitory effect on an M3 receptor, resulting in a significant increase in the IC₅₀ ratio of M3/M2. In addition, the benzodiazepine derivative has specific pharmacological properties, can effectively treat myopia without affecting pupil size, and facilitates improvement of treatment specificity and patient comfort.

## Description

### TECHNICAL FIELD

The present disclosure pertains to the technical field of chemical pharmaceuticals, relates to a medicament for preventing and/or treating myopia and use thereof, and particularly relates to an Al-designed benzodiazepine derivative and use thereof.

### BACKGROUND

Myopia, a common state of refractive abnormality that manifests as a decline in distance vision, has become a major public health problem in modern society (P. N. Baird et al., Nature Reviews, 2020, 6(99):1-20). The trend of myopia becoming increasingly prevalent at younger ages is closely associated with the widespread use of video devices such as computers, mobile phones, and tablets, resulting in a significant increase in screen time, which poses a serious threat to the visual health of children and adolescents.

According to the study by P. N. Baird et al. in Nature Reviews in 2020 and the World Report on Vision released by WHO in 2020, the global number of myopes has reached 2.6 billion. According to the *White Paper on Eye Health in China,* the overall incidence rate of myopia in children and adolescents in China is 53.6%, and the overall incidence rate of myopia in university students exceeds 90%.

After the development of myopia, the balance between accommodation and convergence is disrupted, resulting in symptoms of myopia such as double vision or blurred vision, eyeball swelling and pain, headache, nausea, eye strain, and dryness. Myopia can be classified into simple myopia and pathological myopia according to the disease progression and pathological changes. Simple myopia generally does not cause changes in the fundus, while pathological myopia may be accompanied by symptoms such as exophthalmos, deep anterior chamber, large pupil and slow reaction, and narrow palpebral fissure, and increases the risk of severe eye diseases such as macular degeneration and retinal detachment, further seriously impairing vision (Haarman A. et al., Invest Ophthalmol Vis Sci, 2020, 61(4): 49).

At present, utilizing drugs in prevention and control is an effective means for controlling myopia (J. H. Huang et al., American Academy of Ophthalmology, 2016, 123(4):697-708). It is indicated in the study by Walline J.J. et al. in 2020 that atropine is an effective drug for preventing myopia and/or inhibiting the progression of myopia (Walline J.J. et al., Cochrane Database Syst Rev, 2020, 1: D4916). However, in the study by Joachimsen L. et al. in 2021, when atropine was used for treatment, adverse reactions such as mydriasis and changes in accommodation ability were observed after using 0.01%, 0.025%, and 0.05% atropine (Joachimsen L. et al., Int Ophthalmol, 2021, 41(6): 2001-2008).

In conclusion, there is an urgent need for the prevention and control of myopia, and the existing drugs have a tradeoff between effectiveness and safety.

### SUMMARY

The objective of the present disclosure is to develop a new treatment strategy, so as to provide a safer and more effective solution for the prevention and/or treatment of myopia.

In order to achieve the objective described above, the present disclosure provides an Al-designed benzodiazepine derivative having a structure of formula I: 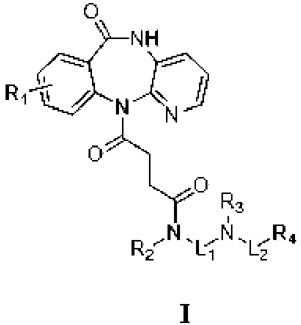
wherein R₁ is any one selected from H, deuterium, halogen, and C₁-C₃ alkoxy;
R₂ and R₃ are each independently selected from H, deuterium, and C₁-C₅ alkyl;
R₄ is polycyclic cycloalkyl or polycyclic heterocyclyl;
L₁ is -(CH₂)ₘ-, wherein m is an integer from 1 to 3;
L₂ is a single bond or -(CH₂)ₙ-, wherein n is 1 or 2.

Optionally, L₂ is a single bond, and R₃ and R₄, together with the N to which they are linked, may form a C₄-C₂₀ polycyclic heterocyclic structure.

Optionally, R₂ and one methylene group in L₁, together with the N to which they are linked, may form a C₄-C₇ heterocyclic structure.

Optionally, the benzodiazepine derivative has a structure of formula II: 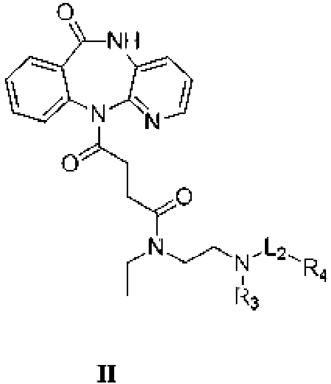
wherein R₃ is any one selected from H, deuterium, and C₁-C₅ alkyl;
R₄ is polycyclic cycloalkyl;
L₂ is a single bond or -(CH₂)ₙ-, wherein n is 1 or 2.

Optionally, L₂ is a single bond, and R₃ and R₄, together with the N to which they are linked, may form a C₇-C₂₀ heteropolycyclic structure.

Optionally, the benzodiazepine derivative comprises any one selected from the following:

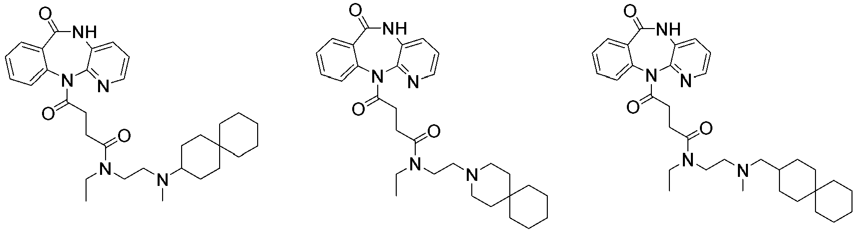

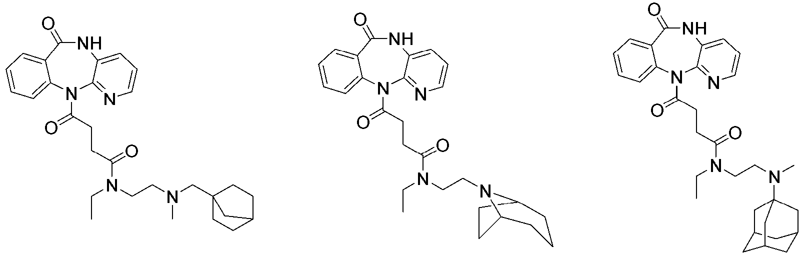

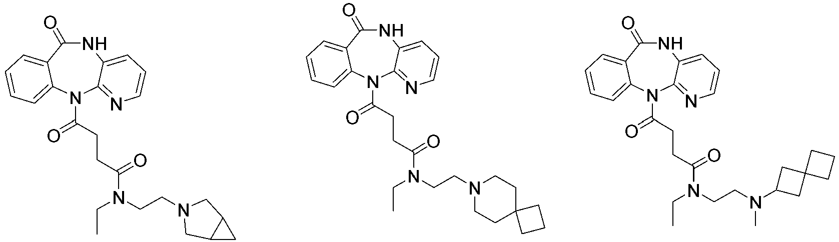

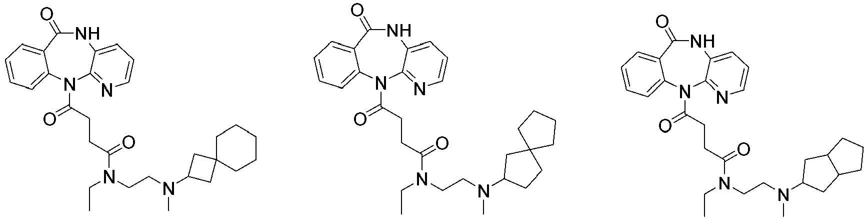

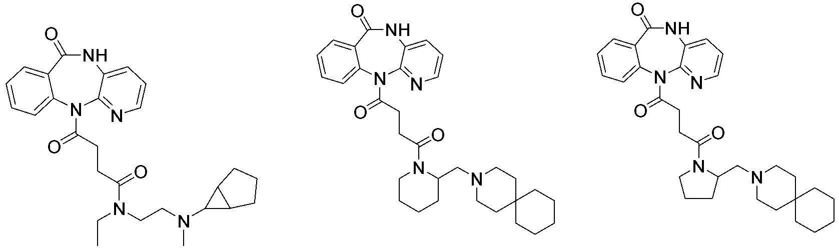

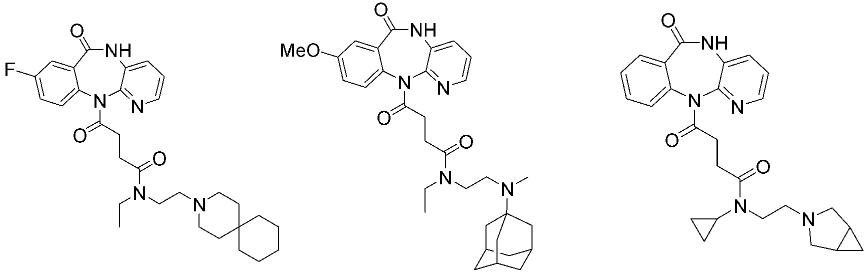

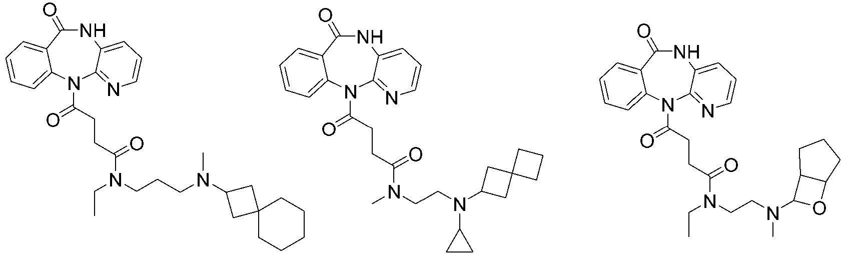

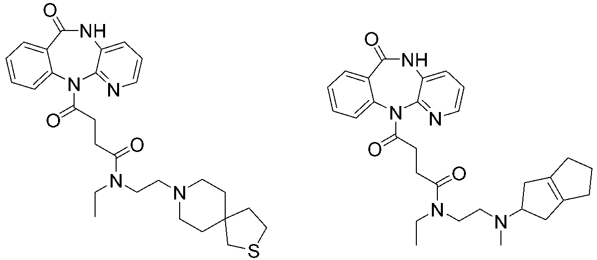

The present disclosure further provides a pharmaceutical composition comprising the AI-designed benzodiazepine derivative described above as an active ingredient.

Optionally, the pharmaceutical composition further comprises a pharmaceutical adjuvant.

The present disclosure further provides use of an AI-designed benzodiazepine derivative in the manufacture of a medicament for preventing myopia and/or inhibiting the progression of myopia.

Optionally, the myopia comprises at least one selected from axial myopia, refractive myopia, pseudomyopia, pathological myopia, simple myopia, extreme myopia, severe myopia, high myopia, moderate myopia, low myopia, myopia complicated by glaucoma, myopia at risk of developing glaucoma, or myopia with high intraocular pressure.

Compared with the prior art, the technical solutions of the present disclosure have at least the following beneficial effects:
Experiments prove that the AI-designed benzodiazepine derivative provided by the present disclosure has high selectivity for M2 and a significant inhibitory effect on human M2 cells, but exhibits little inhibition against human M3, and the IC₅₀ ratio of M3/M2 is much higher than that of the existing drug AFDX-116 for myopia in the clinical stage. Therefore, the benzodiazepine derivative of the present disclosure is expected to prevent the development of myopia and/or inhibit the progression of myopia, without causing the side effect of mydriasis.

### BRIEF DESCRIPTION OF THE DRAWING

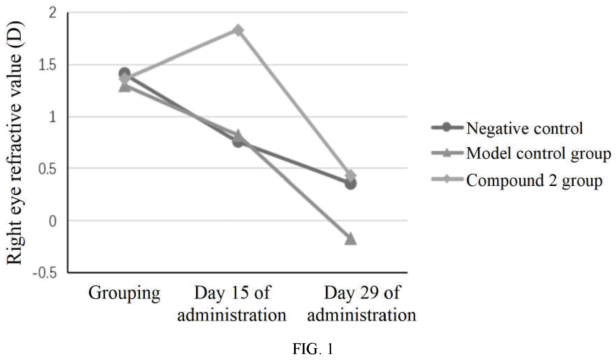

FIG. 1 shows a schematic diagram of the effect of compound 2 of the present disclosure on the diopter of a myopia guinea pig model.

### DETAILED DESCRIPTION

The technical solutions of the present disclosure will be clearly and completely described below with reference to the accompanying drawing, and it is obvious that the described examples are some of the examples of the present disclosure, but not all of them. Based on the examples of the present disclosure, all other examples obtained by those of ordinary skill in the art without creative efforts shall fall within the protection scope of the present disclosure.

The M2 receptors are mainly distributed on the ciliary muscle and retina of the eye and are involved in regulating the focusing and refractive states of the eye. Barathi et al. (BarathiV.A. et al. Dis. Model. Mech. 2013, 6 (5): 1146-1158) knocked out the muscarinic M2 receptor gene in mice. After 4-8 weeks of minus-lens induction, mice in the control group showed a significant increase in the axial length, lens thickness, and vitreous chamber depth, exhibiting symptoms of myopia. However, M2 gene-knockout mice did not exhibit symptoms of myopia. In M2 receptor gene-knockout mice, type I collagen in the sclera was increased, while type V collagen was decreased, indicating enhanced scleral fibrosis, which in turn inhibited axial elongation of the eye. This suggests that myopia can be treated or prevented by inhibiting the activity of the M2 receptor. This document also discloses two examples of selective M2 receptor inhibitors for inhibiting the progression of myopia, one of which is AFDX-116.

M3 receptors, muscarinic type 3 receptors, are mainly distributed on the sphincter pupillae of the eye. The M3 receptors are associated with the contraction of the sphincter pupillae and affect the size of the pupil. When these receptors are activated, they cause the sphincter pupillae to contract, thereby constricting the pupil. This process is called miosis. Conversely, when the activity of the M3 receptor is reduced or inhibited, the constriction of the sphincter pupillae is attenuated, causing the pupil to dilate, i.e., mydriasis occurs.

Therefore, in the treatment or prevention of myopia according to the present disclosure, in order to reduce mydriasis, the selectivity of the drug for the M2 receptor is considered to be improved as much as possible, and it is desirable that the drug mainly acts on the M2 receptor and has a relatively small effect on other muscarinic receptors (e.g., the M3 receptor), that is, a higher IC₅₀ ratio of M3/M2 is better.

In the development of the latest AI technology, artificial intelligence can be used for the discovery, screening, and optimization of small molecule drugs. Novel therapeutic drugs are invented by integrating AI computing tools, databases (e.g., CAS compound libraries), and receptor-ligand complex interaction information into efficient molecule/atom training sets and AI digital workflows, and then combining with BT technology (bioinformatics, *in vitro* target cell biological activity assay, etc.).

It is well known that during drug development, it takes a lot of time (usually 5-6 years or longer) for a traditional small molecule compound (drug) to go from a hit compound to a lead compound and then to a candidate compound, while artificial intelligence (AI Drug Discovery & Design, AIDD)-driven drug development can perform data cross-comparison, molecular docking, molecular dynamics simulation, and the like, in a relatively short time (usually only 3-5 months), thereby accelerating the screening or *de novo* design to generate novel compounds, the core value of which is reflected in ground-breaking innovation and efficiency improvement.

Based on relatively clear mechanisms (e.g., the Schrödinger equation and the Gibbs free energy change), the AI can search a plurality of broader, comprehensive, and different compound and proteomics databases, thereby quickly completing searches and cross-comparison, and saving a large amount of investment originally used for wet experiments. The wet experiment data can be fed back to the AI for iterative optimization of compound structures.

According to the present disclosure, a computational structural biology technology platform based on an AI (dry experiment) + BT (wet experiment) interactive fusion research and development mode that is built in-house is combined with a variety of advanced algorithms in pharmaceutical chemistry and quantum chemistry, as well as deep learning, natural language processing, and various pre-trained neural network framework models to generate a novel molecular structure with expected functional properties, and then molecular modeling, molecular docking, molecular dynamics simulation, and the like are performed according to the target-drug related binding interface to obtain an optimal receptor-ligand binding free energy function, association constant, dissociation constant, and the like, thereby optimizing and screening candidate compounds with the best *in vitro* cell activity and the most suitable comprehensive evaluation. Meanwhile, through deep analysis of disease/drug correlation networks, AI servers, and workstations, deep learning for drugs and multi-threaded collaborative simulations can be performed independently and simultaneously, and AI computing power clusters can also be formed for big data processing of libraries and candidate compounds, so as to deeply mine libraries, structure-pharmacophore relationships (QSAR), and novel drug targets, thereby saving innovative drug development costs and shortening research and development time. Therefore, a brand new research and development path for new drugs, which is characterized by "a combination of dry and wet experiments and seamless integration", is formed and is expected to become a scientific paradigm for future innovative drug research and development. This brings a brand new breakthrough idea and experimental innovation to the research and development process of past drugs, which relies heavily on wet experiments and is time-consuming, labor-intensive, and cumbersome.

Through multiple rounds of iterative dry-wet closed-loop experimental screening and verification, a series of benzodiazepine derivatives of the present disclosure are finally obtained, having a structure of formula I: 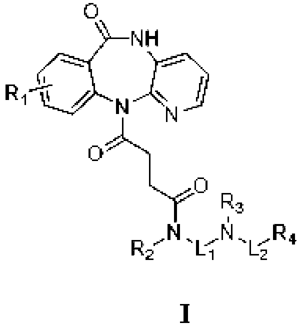 wherein R₁ is any one selected from H, deuterium, halogen, and C₁-C₃ alkoxy; R₂ and R₃ are each independently selected from H, deuterium, and C₁-C₅ alkyl; R₄ is polycyclic cycloalkyl or polycyclic heterocyclyl; L₁ is -(CH₂)ₘ-, wherein m is an integer from 1 to 3; L₂ is a single bond or -(CH₂)ₙ-, wherein n is 1 or 2.

Optionally, L₂ is a single bond, and R₃ and R₄, together with the N to which they are linked, may form a C₄-C₂₀ polycyclic heterocyclic structure.

Optionally, R₂ and one methylene group in L₁, together with the N to which they are linked, may form a C₄-C₇ heterocyclic structure.

Optionally, the benzodiazepine derivative has a structure of formula II: 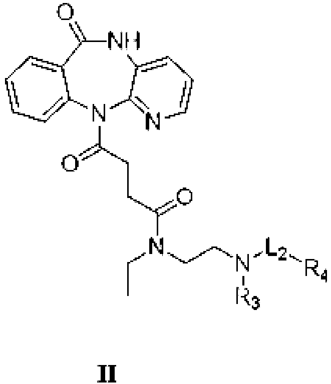 wherein R₃ is any one selected from H, deuterium, and C₁-C₅ alkyl; R₄ is polycyclic cycloalkyl; L₂ is a single bond or -(CH₂)ₙ-, wherein n is 1 or 2.

The present disclosure further provides a pharmaceutical composition comprising the AI-designed benzodiazepine derivative described above as an active ingredient, and a pharmaceutical adjuvant.

The present disclosure further provides use of an AI-designed benzodiazepine derivative in the manufacture of a medicament for preventing myopia and/or inhibiting the progression of myopia.

### Definitions of Terms

The "single bond" described herein means that R₄ is directly linked to the N linked to R₃, i.e., L2 is a chemical bond linking N and R₄.

The "halogen" described herein is fluorine, chlorine, bromine, and iodine.

The "C₁-C₃ alkoxy" described herein refers to a group formed by linking an alkyl group having 1 to 3 carbon atoms to an oxygen atom, wherein the oxygen atom has the ability of free bonding, such as methoxy, ethoxy, and propoxy.

The "C₁-C₅ alkyl" described herein refers to a linear, branched, or cyclic alkyl group having 1 to 5 carbon atoms. Examples of the linear alkyl include methyl, ethyl, n-propyl, n-butyl, and n-pentyl; examples of the branched alkyl include, but are not limited to, isopropyl, isobutyl, *sec*-butyl, *tert*-butyl, neopentyl, and isopentyl; examples of the cycloalkyl include, but are not limited to, cyclopropyl, cyclobutyl, and cyclopentyl.

The "polycyclic cycloalkyl" described herein refers to a saturated or partially unsaturated polycyclic substituent. The polycyclic cycloalkyl ring contains 4-20 carbon atoms, preferably 7-12 carbon atoms. The polycyclic cycloalkyl includes a spiro ring, a fused ring, and a bridged ring.

The "spiro ring" described herein refers to a 5- to 20-membered polycyclic group in which monocyclic rings share one carbon atom (referred to as the spiro atom), and the group may contain one or more double bonds. It is preferably 6- to 14-membered, and is more preferably 7- to 10-membered (e.g., 7-, 8-, 9-, or 10-membered). According to the number of spiro atoms shared between rings, spirocycloalkyl may be monospirocycloalkyl, bispirocycloalkyl, or polyspirocycloalkyl, preferably monospirocycloalkyl and bispirocycloalkyl, and more preferably 3-membered/5-membered, 3-membered/6-membered, 4-membered/4-membered, 4-membered/5-membered, 4-membered/6-membered, 5-membered/5-membered, or 5-membered/6-membered monospirocycloalkyl. Non-limiting examples of spirocycloalkyl include: at least one selected from 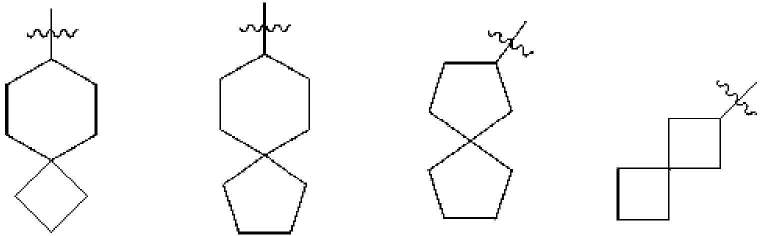 or

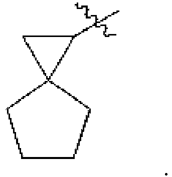

The "fused ring" described herein refers to a 5- to 20-membered all-carbon polycyclic group in which each ring shares a pair of adjacent carbon atoms with the other rings in the structure, wherein one or more rings may contain one or more double bonds. It is preferably 6- to 14-membered, and is more preferably 7- to 10-membered (e.g., 7-, 8-, 9-, or 10-membered). According to the number of constituent rings, it may be bicyclic, tricyclic, tetracyclic, or polycyclic fused cycloalkyl, preferably bicyclic or tricyclic, and more preferably 5-membered/5-membered or 5-membered/6-membered bicycloalkyl. Non-limiting examples of fused cycloalkyl include: at least one selected from 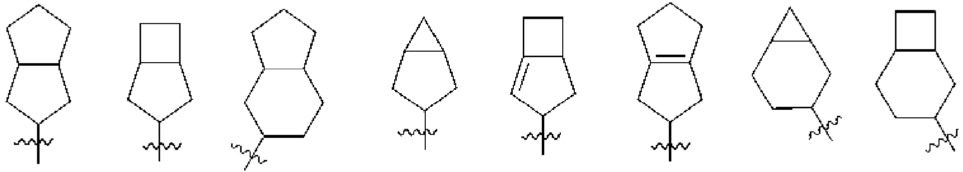 or

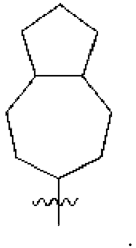

The "bridged ring" described herein refers to a 5- to 20-membered all-carbon polycyclic group in which any two rings share two carbon atoms that are not directly linked, and the group may contain one or more double bonds. It is preferably 6- to 14-membered, and is more preferably 7- to 10-membered (e.g., 7-, 8-, 9-, or 10-membered). According to the number of constituent rings, it may be bicyclic, tricyclic, tetracyclic, or polycyclic bridged cycloalkyl, preferably bicyclic, tricyclic, or tetracyclic, and more preferably bicyclic or tricyclic. Non-limiting examples of bridged cycloalkyl include: at least one selected from

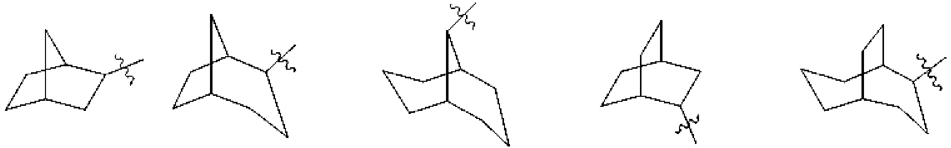

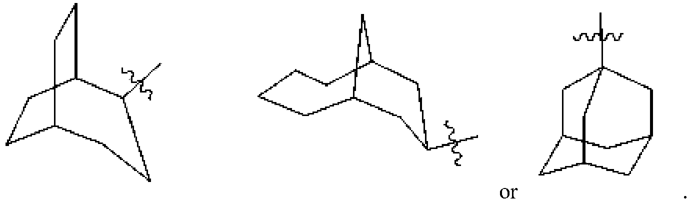

The polycyclic cycloalkyl may be substituted or unsubstituted. When it is substituted, the substituent may be substituted at any available point of attachment, and the substituent is preferably one or more substituents independently optionally selected from halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The "polycyclic heterocyclyl" described herein refers to a saturated or partially unsaturated polycyclic hydrocarbon substituent containing 4 to 20 ring atoms, wherein one or more ring atoms are heteroatoms selected from nitrogen, oxygen, and sulfur; the sulfur may optionally be substituted with oxo (i.e., to form sulfoxide or sulfone), but a ring moiety -O-O-, -O-S-, or -S-S- is not included; and the remaining ring atoms are carbon atoms. The polycyclic heterocyclyl preferably contains 7 to 12 ring atoms, of which 1 to 4 (e.g., 1, 2, 3, and 4) are heteroatoms. The polycyclic heterocyclyl includes spiroheterocyclyl, fused heterocyclyl, and bridged heterocyclyl.

The "spiroheterocyclyl" described herein refers to a 5- to 20-membered polycyclic heterocyclyl group in which monocyclic rings share one atom (referred to as the spiro atom), wherein one or more ring atoms are heteroatoms selected from nitrogen, oxygen, and sulfur; the sulfur may optionally be substituted with oxo (i.e., to form sulfoxide or sulfone); and the remaining ring atoms are carbon atoms. It may contain one or more double bonds. It is preferably 6- to 14-membered. According to the number of spiro atoms shared between rings, spiroheterocyclyl may be monospiroheterocyclyl, bispiroheterocyclyl, or polyspiroheterocyclyl, preferably monospiroheterocyclyl and bispiroheterocyclyl, and more preferably 4-membered/4-membered, 4-membered/5-membered, 4-membered/6-membered, 5-membered/5-membered, or 5-membered/6-membered monospiroheterocyclyl. Non-limiting examples of spiroheterocyclyl include: at least one selected from 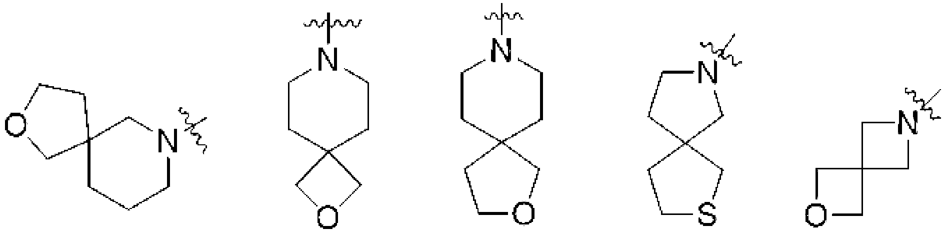 or

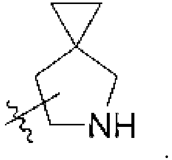

The "fused heterocyclyl" described herein refers to a 4- to 20-membered polycyclic heterocyclyl group in which each ring shares a pair of adjacent atoms with the other rings in the structure, and one or more rings may contain one or more double bonds, wherein one or more ring atoms are heteroatoms selected from nitrogen, oxygen, and sulfur; the sulfur may optionally be substituted with oxo (i.e., to form sulfoxide or sulfone); and the remaining ring atoms are carbon atoms. It is preferably 6- to 14-membered, and is more preferably 7- to 10-membered (e.g., 7-, 8-, 9-, or 10-membered). According to the number of constituent rings, it may be bicyclic, tricyclic, tetracyclic, or polycyclic fused heterocyclyl, preferably bicyclic or tricyclic, and more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused heterocyclyl. Non-limiting examples of fused heterocyclyl include: at least one selected from 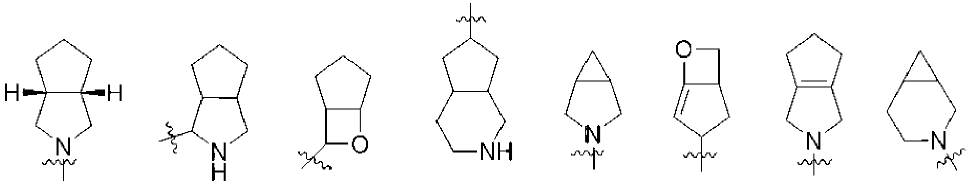 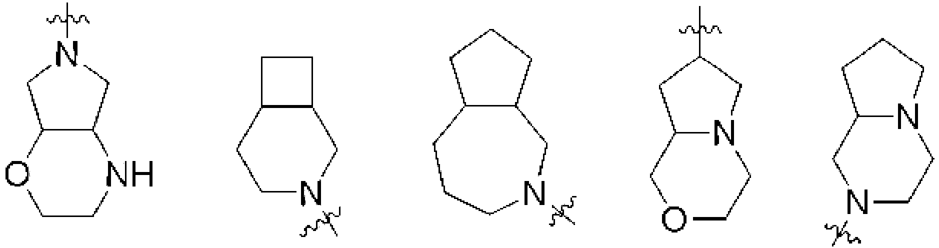 or

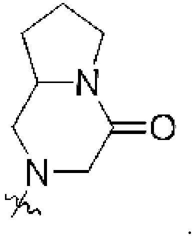

The "bridged heterocyclyl" described herein refers to a 5- to 14-membered polycyclic heterocyclyl group in which any two rings share two atoms that are not directly linked, and the group may contain one or more double bonds, wherein one or more ring atoms are heteroatoms selected from nitrogen, oxygen, and sulfur; the sulfur may optionally be substituted with oxo (i.e., to form sulfoxide or sulfone); and the remaining ring atoms are carbon atoms. It is preferably 6- to 14-membered, and is more preferably 7- to 10-membered (e.g., 7-, 8-, 9-, or 10-membered). According to the number of constituent rings, it may be bicyclic, tricyclic, tetracyclic, or polycyclic bridged heterocyclyl, preferably bicyclic, tricyclic, or tetracyclic, and more preferably bicyclic or tricyclic. Non-limiting examples of bridged heterocyclyl include: at least one selected from

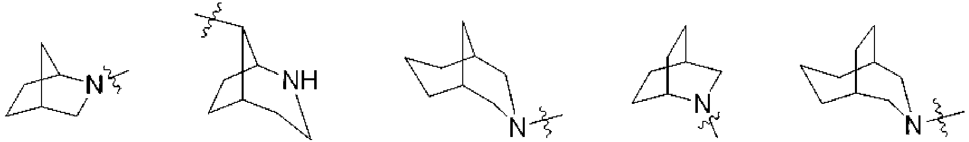

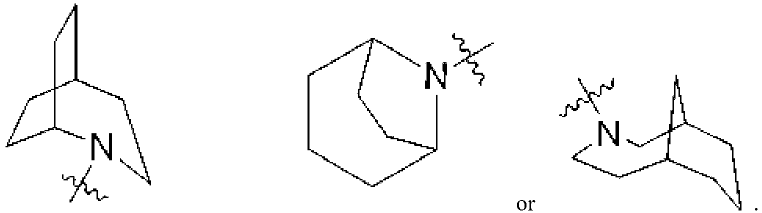

The polycyclic heterocyclyl may be substituted or unsubstituted. When it is substituted, the substituent may be substituted at any available point of attachment, and the substituent is preferably one or more substituents independently optionally selected from hydrogen, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The "C₄-C₇ heterocyclic ring" described herein refers to a saturated or partially unsaturated monocyclic hydrocarbon substituent containing 4 to 7 ring atoms in total, wherein one or two ring atoms are heteroatoms selected from nitrogen, oxygen, and sulfur; the sulfur may optionally be substituted with oxo (i.e., to form sulfoxide or sulfone); and the remaining ring atoms are carbon atoms.

The "substituted" described herein means that an organic group as defined herein (containing one or more bonds to a hydrogen atom) is substituted with one or more bonds to a non-hydrogen atom or atomic group, and the non-hydrogen atom or atomic group is a substituent.

The present disclosure further provides an isotopically labeled compound of the compound of the present disclosure. The "isotopically labeled compound" of the compound of the present disclosure or the "isotopically labeled" compound of the present disclosure refers to a compound described herein in which one or more atoms are replaced by an isotope atom having an atomic mass or mass number different from the atomic mass or mass number of those usually found in nature (i.e., naturally occurring). Suitable radionuclides may include, but are not limited to, 2H (deuterium, also written as D), 3H (tritium, also written as T), 11C, 13C, 14C, 13N, 15N, 150, 170, 180, 18F, 35S, 36Cl, 82Br, 75Br, 76Br, 77Br, 123I, 124I, 125I, and 131I. The type of radioisotope contained in the isotopically labeled compound will depend on the specific application of the isotopically labeled compound. For example, for labeling and competition assays of IDO enzymes *in vitro,* compounds comprising 3H, 14C, 82Br, 125I, 131I, or 35S are generally most useful. For isotope imaging applications, 11C, 18F, 125I, 123I, 124I, 131I, 75Br, 76Br, or 77Br is generally the most useful.

Methods known in the art for labeling organic compounds with radioisotopes are also applicable to the compound of the present disclosure.

When administered as a medicament, the compound may be administered in the form of a pharmaceutical composition. Therefore, in another aspect, the present application provides a pharmaceutical composition comprising the compound or the pharmaceutically acceptable salt thereof, and at least one pharmaceutically acceptable carrier or excipient.

The term "composition" used herein refers to a product comprising the compound or the pharmaceutically acceptable salt thereof disclosed in the present application as a specific active ingredient, and any other product that is directly or indirectly combined with the active ingredient.

Generally, the pharmaceutical composition comprises at least one pharmaceutically acceptable carrier or excipient. The term "pharmaceutically acceptable" means that the carrier or excipient is compatible with the other ingredients in the formulation and not deleterious to subjects. The carrier described herein refers to a substance for improving the selectivity, effectiveness, and/or safety of the drug during delivery. The carrier is mainly used to control drug release, and may also be used to improve the pharmacokinetic properties, especially bioavailability, of the drug. The excipient refers to substances other than the active ingredient in the pharmaceutical formulation, which are mainly used for long-term stability, for filling solid formulations (thus, also commonly referred to as "fillers"), or for enhancing product efficacy (e.g., promoting absorption, reducing viscosity, or increasing solubility).

The "myopia" described herein includes at least one selected from axial myopia, refractive myopia, pseudomyopia, pathological myopia, simple myopia, extreme myopia, severe myopia, high myopia, moderate myopia, low myopia, myopia complicated by glaucoma, myopia at risk of developing glaucoma, or myopia with high intraocular pressure. Among them, the extreme myopia, severe myopia, high myopia, moderate myopia, and low myopia are categories of myopia classified according to different diopters. Herein, the extreme myopia is characterized by a diopter of -10.00 D or less; the severe myopia is characterized by a diopter between -6.00 D and -9.99 D; the high myopia is characterized by a diopter between -4.00 D and -5.99 D; the moderate myopia is characterized by a diopter between -2.00 D and -3.99 D; and the low myopia is characterized by a diopter between -0.50 D and -1.99 D.

The technical solutions of the present disclosure will be clearly and completely described below with reference to the examples of the present disclosure, and it is obvious that the described examples are some of the examples of the present disclosure, but not all of them. Based on the examples of the present disclosure, all other examples obtained by those of ordinary skill in the art without creative efforts shall fall within the protection scope of the present disclosure.

Unless otherwise indicated, the experimental methods used in the following examples are all performed under conventional conditions or conditions recommended by the manufacturer. The materials, reagents, and the like used in the following examples can be commercially available, unless otherwise indicated.

The full names represented by the abbreviations of the reagents used in the examples are as follows:
TEA: triethylamine
DMSO: dimethyl sulfoxide
TFA: trifluoroacetic acid
HATU: *O*-(7-azabenzotriazol-1-yl)-*N*,*N*,*N'*,*N'*-tetramethyluronium hexafluorophosphate
DIEA: N,N-diisopropylethylamine.

### Example 1: Preparation of Compound 1

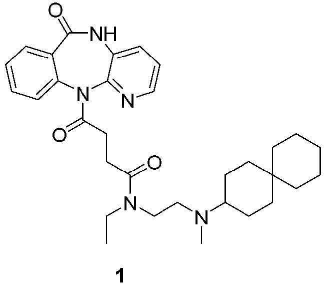

### Synthetic route of compound 1:

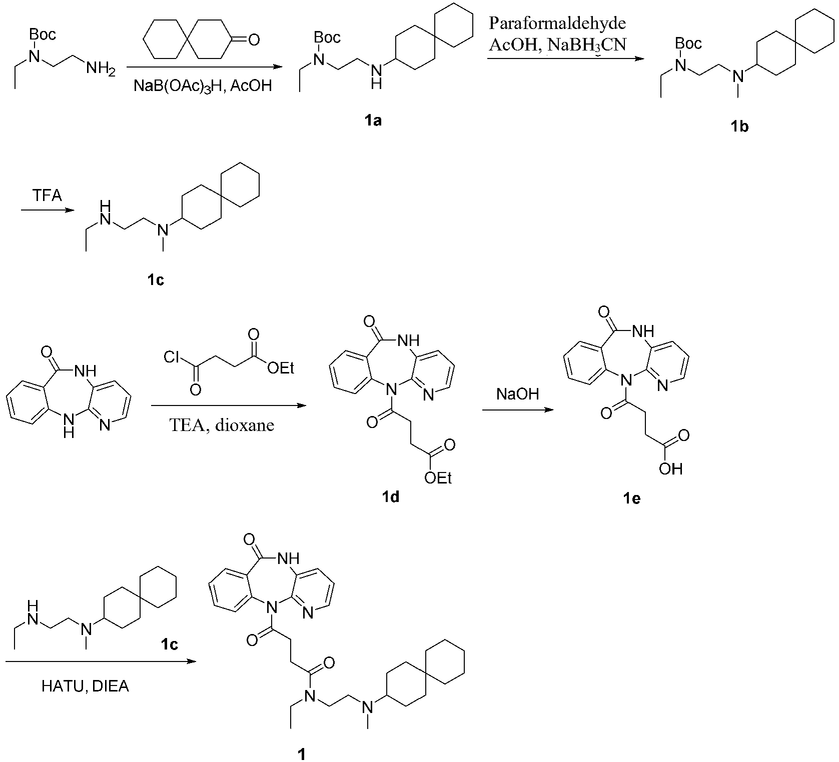

### Step 1: Preparation of intermediate 1a

tert-Butyl (2-aminoethyl)(ethyl)carbamate (226 mg, 1.20 mmol) was dissolved in dichloroethane (10 mL), and spiro[5.5]undecan-3-one (200 mg, 1.20 mmol) and acetic acid (72 mg, 1.20 mmol) were added. After the resulting system was stirred at room temperature for 1 h under nitrogen atmosphere, sodium triacetoxyborohydride (1.27 g, 6.01 mmol) was added, and the system was reacted at room temperature for another 3 h.

A saturated aqueous sodium bicarbonate solution (20 mL) was added to quench the reaction. The aqueous phase was extracted three times with dichloromethane (30 mL). The organic phases were combined, washed twice with brine (50 mL), dried over anhydrous sodium sulfate, filtered, and concentrated to dryness by rotary evaporation.

The resulting residue was purified by silica gel column chromatography (eluent: dichloromethane/methanol = 20/1) to give intermediate 1a (350 mg, yield: 85.9%).

LCMS: [Ms+H]⁺ = 339.4.

### Step 2: Preparation of intermediate 1b

Intermediate 1a (350 mg, 1.03 mmol) was dissolved in methanol (10 mL), and then paraformaldehyde (310 mg, 10.34 mmol), acetic acid (310 mg, 5.17 mmol), and sodium triacetoxyborohydride (260 mg, 414 mmol) were added. The mixture was reacted at room temperature for 16 h.

A saturated aqueous sodium bicarbonate solution (20 mL) was added to quench the reaction. The aqueous phase was extracted three times with dichloromethane (30 mL). The organic phases were combined, washed twice with brine (50 mL), dried over anhydrous sodium sulfate, filtered, and concentrated to dryness by rotary evaporation.

The resulting residue was purified by silica gel column chromatography (eluent: dichloromethane/methanol = 20/1) to give intermediate 1b (320 mg, yield: 87.8%).

LCMS: [Ms+H]⁺ = 353.3.

### Step 3: Preparation of intermediate 1c

Intermediate 1b (160 mg, 0.45 mmol) was dissolved in dichloromethane (3 mL), and trifluoroacetic acid (1 mL) was added at 0 °C. The mixture was reacted at room temperature for 3 h.

The reaction solution was concentrated to dryness by rotary evaporation, and the resulting residue was azeotroped three times with dichloromethane (30 mL).

The resulting crude product was directly used in the next step without further purification.

LCMS: [Ms+H]⁺ = 253.2.

### Step 4: Preparation of intermediate 1d

5,11-Dihydro-6H-benzopyrido[3,2-b][1,4]diazepin-6-one (2 g, 12 mmol) and triethylamine (1.6 mL, 6 mmol) were dissolved in 1,4-dioxane (40 mL). Subsequently, the mixture was heated to 80 °C, and ethyl 4-chloro-4-oxobutanoate (2.4 g, 11.3 mmol) was added. The mixture was reacted at 100 °C for 4 h under nitrogen atmosphere.

Water (30 mL) was added to quench the reaction. The mixture was then extracted three times with ethyl acetate (20 mL). The organic phases were combined, washed three times with brine (20 mL), dried over anhydrous sodium sulfate, and concentrated to dryness by rotary evaporation.

The resulting residue was purified by silica gel column chromatography (eluent: dichloromethane/methanol = 50/1) to give intermediate 1d (1.1 g, yield: 27%).

LCMS: [Ms+H]⁺ = 340.2.

### Step 5: Preparation of intermediate 1e

Intermediate 1d (155 mg, 0.46 mmol) was dissolved in ethanol (3 mL), and a 1 N aqueous sodium hydroxide solution (1.5 mmol, 1.5 mL) was added. The mixture was reacted at room temperature for 1 h.

A 1 N aqueous hydrochloric acid solution (1.5 mL) was added for neutralization. The reaction solution was then concentrated to dryness by rotary evaporation, followed by the addition of N,N-dimethylformamide. The mixture was filtered to give a solution of intermediate 1e (140 mg) in N,N-dimethylformamide, which was directly used in the next step.

LCMS: [Ms+H]⁺ = 312.0.

### Step 6: Preparation of compound 1

Intermediate 1e (140 mg, 0.45 mmol) and intermediate 1c (114 mg, 0.45 mmol) were dissolved in N,N-dimethylformamide (5 mL), and N,N-diisopropylethylamine (292 mg, 2.26 mmol) and *O*-(7-azabenzotriazol-1-yl)-*N*,*N*,*N'*,*N'*-tetramethyluronium hexafluorophosphate (206 mg, 0.54 mmol) were added sequentially. The mixture was reacted at room temperature for 16 h under nitrogen atmosphere.

The reaction system was diluted with water (20 mL) and extracted three times with dichloromethane (20 mL). The organic phases were combined, washed with brine (30 mL), dried over anhydrous sodium sulfate, and concentrated.

The resulting residue was purified by Pre-HPLC to give compound 1 (56.56 mg, yield: 23.0%).

LCMS: [Ms+H]⁺ = 546.4.

¹H NMR (400 MHz, CDCl₃) *δ* 8.34 (s, 1H), 7.91 (d, J = 7.1 Hz, 1H), 7.78 - 7.57 (m, 2H), 7.52 (d, J = 7.2 Hz, 1H), 7.42 (t, J = 7.0 Hz, 1H), 7.35 (s, 1H), 4.60 (s, 3H), 4.12 - 3.62 (m, 1H), 3.59 - 3.28 (m, 3H), 3.24 - 3.00 (m, 3H), 2.96 - 2.64 (m, 5H), 2.61-2.25 (m, 2H), 2.00 - 1.67 (m, 3H), 1.65 - 1.47 (m, 2H), 1.46 - 1.25 (m, 8H), 1.16 (t, J = 7.0 Hz, 3H), 1.09 - 0.85 (m, 2H).

### Example 2: Preparation of Compound 2

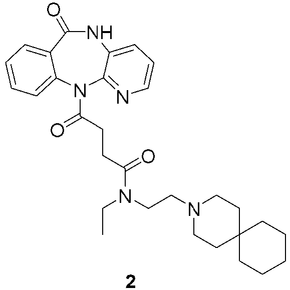

### Synthetic route of compound 2:

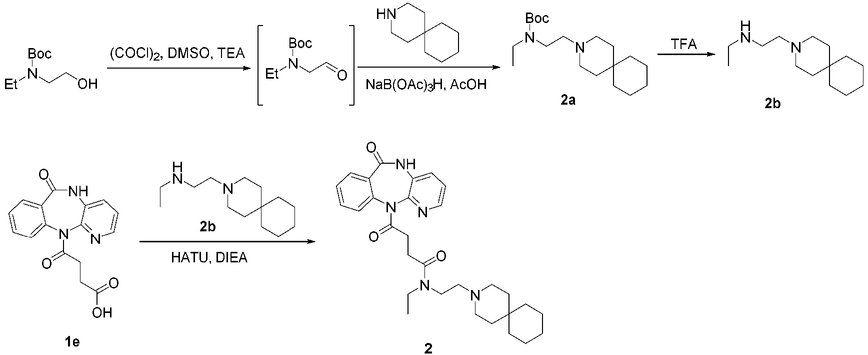

### Step 1: Preparation of intermediate 2a

Oxalyl chloride (563 mg, 4.44 mmol) was dissolved in dichloromethane (15 mL), and then a solution of dimethyl sulfoxide (421 mg, 5.39 mmol) in dichloromethane (3 mL) was added dropwise at -78 °C. After the resulting system was stirred at -78 °C for 25 min under nitrogen atmosphere, a solution of tert-butyl ethyl(2-hydroxyethyl)carbamate (600 mg, 3.17 mmol) in dichloromethane (3 mL) was added dropwise. After the resulting mixture was stirred at -78 °C for 25 min under nitrogen atmosphere, a solution of triethylamine (1.12 g, 11.10 mmol) in dichloromethane (3 mL) was added dropwise, and then the mixture was heated to room temperature and reacted for 2 h. Subsequently, the reaction solution was cooled to 0 °C, and 3-azaspiro[5.5]undecane (202 mg, 1.32 mmol), acetic acid (1.11 g, 18.45 mmol), and sodium triacetoxyborohydride (978 mg, 4.61 mmol) were added. The mixture was reacted at room temperature for 1 h.

A saturated aqueous sodium bicarbonate solution (10 mL) was added to quench the reaction. The aqueous phase was extracted three times with dichloromethane (30 mL). The organic phases were combined, washed twice with brine (50 mL), dried over anhydrous sodium sulfate, filtered, and concentrated to dryness by rotary evaporation.

The resulting residue was purified by silica gel column chromatography (eluent: petroleum ether/ethyl acetate = 1/1) to give intermediate 2a (330 mg, yield: 80.6%).

LCMS: [Ms+H]⁺ = 325.5.

### Step 2: Preparation of intermediate 2b

Intermediate 2b was prepared from intermediate 2a (160 mg, 0.49 mmol) according to the procedure of step 3 in Example 1 to give a crude product (110 mg), which was directly used in the next step.

LCMS: [Ms+H]⁺ = 225.5.

### Step 3: Preparation of compound 2

Compound 2 was prepared from intermediate 1e (110 mg, 0.49 mmol) and intermediate 2b (153 mg, 0.49 mmol) according to the procedure of step 6 in Example 1, with a yield of 15.9%.

LCMS: [Ms+H]⁺ = 518.6.

¹H NMR (400 MHz, CDCl₃) *δ* 9.41 (s, 1H), 8.34 (s, 1H), 7.89 (d, J = 7.7 Hz, 1H), 7.75 - 7.56 (m, 2H), 7.51 (d, J = 7.1 Hz, 1H), 7.46 - 7.30 (m, 2H), 4.05 - 3.67 (m, 2H), 3.56 - 3.13 (m, 6H), 3.03 - 2.74 (m, 4H), 2.74 - 2.62 (m, 1H), 2.62 - 2.48 (m, 1H), 2.32 (s, 6H), 1.89 - 1.62 (m, 4H), 1.37 - 1.21 (m, 4H), 1.13 (t, J = 7.0 Hz, 3H).

### Example 3: Preparation of Compound 3

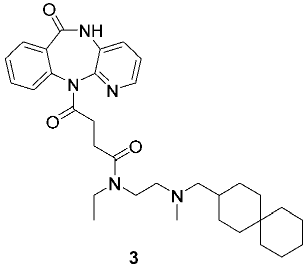

### Synthetic route of compound 3:

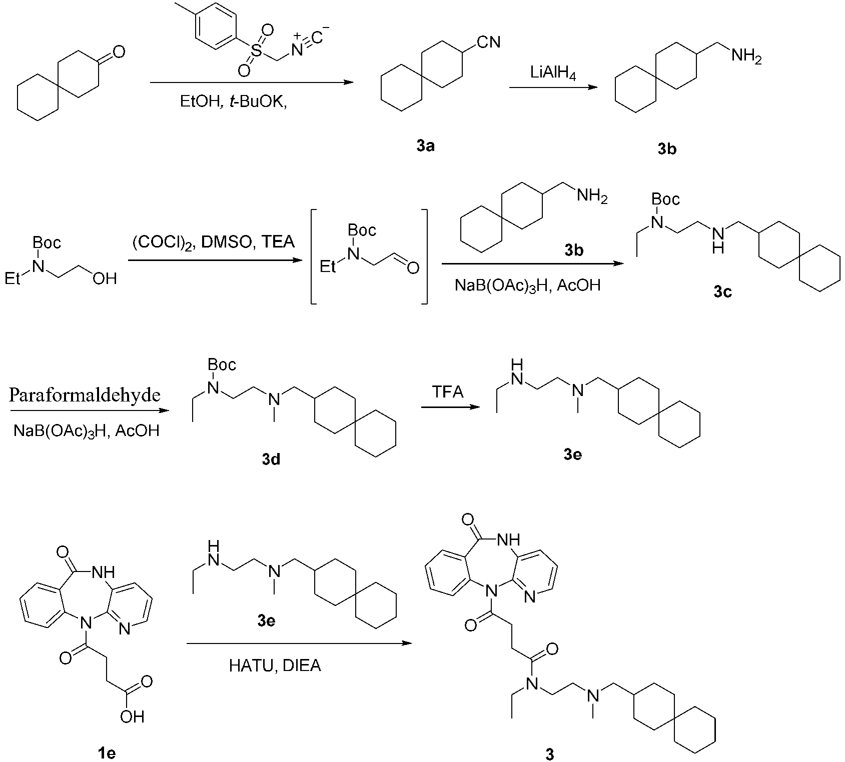

### Step 1: Preparation of intermediate 3a

Spiro[5.5]undecan-3-one (450 mg, 2.71 mmol) was dissolved in ethylene glycol dimethyl ether (10 mL), and then p-toluenesulfonylmethyl isocyanide (687 mg, 3.52 mmol), ethanol (249 mg, 5.41 mmol), and potassium tert-butoxide (729 mg, 6.50 mmol) were added at 0 °C. The resulting system was stirred at 0 °C for 1 h under nitrogen atmosphere and then reacted at room temperature for 3 h.

The reaction solution was concentrated to dryness by rotary evaporation, and then diethyl ether (20 mL) was added. The resulting mixture was stirred for 0.5 h and filtered. This operation was repeated twice. The filtrates were combined and concentrated to dryness by rotary evaporation.

The resulting residue was purified by silica gel column chromatography (eluent: petroleum ether/ethyl acetate = 5/1) to give intermediate 3a (240 mg, yield: 50.0%).

¹H NMR (400 MHz, CDCl₃) *δ* 2.56 (dq, J = 12.6, 4.2 Hz, 1H), 1.91 - 1.66 (m, 4H), 1.66 - 1.54 (m, 2H), 1.50 - 1.10 (m, 12H).

### Step 2: Preparation of intermediate 3b

Intermediate 3a (240 mg, 1.35 mmol) was dissolved in tetrahydrofuran (10 mL), and then a 2.5 N solution of lithium aluminum hydride in tetrahydrofuran (1.08 mL, 2.71 mmol) was added dropwise at 0 °C. The resulting system was stirred at 70 °C for 4 h under nitrogen atmosphere.

A 1 N saturated aqueous sodium hydroxide solution (10 mL) was added to quench the reaction. The aqueous phase was extracted three times with ethyl acetate (30 mL). The organic phases were combined, washed twice with brine (50 mL), dried over anhydrous sodium sulfate, filtered, and concentrated to dryness by rotary evaporation. The crude product was directly used in the next step without purification.

LCMS: [Ms+H]⁺ = 182.2.

### Step 3: Preparation of intermediate 3c

Intermediate 3c was prepared from *tert*-butyl ethyl(2-hydroxyethyl)carbamate (313 mg, 1.65 mmol) and intermediate 3b (240 mg, 1.32 mmol) according to the procedure of step 1 in Example 2, with a yield of 21.4%.

LCMS: [Ms+H]⁺ = 353.3.

### Step 4: Preparation of intermediate 3d

Intermediate 3c (100 mg, 0.28 mmol) was dissolved in methanol (5 mL), and then paraformaldehyde (85 mg, 2.84 mmol), acetic acid (85 mg, 1.42 mmol), and sodium triacetoxyborohydride (71 mg, 1.13 mmol) were added. The mixture was reacted at room temperature for 16 h.

A saturated aqueous sodium bicarbonate solution (20 mL) was added to quench the reaction. The aqueous phase was extracted three times with dichloromethane (30 mL). The organic phases were combined, washed twice with brine (50 mL), dried over anhydrous sodium sulfate, filtered, and concentrated to dryness by rotary evaporation. The resulting residue was intermediate 3d, which was directly used in the next step without purification.

LCMS: [Ms+H]⁺ = 367.3.

### Step 5: Preparation of intermediate 3e

Intermediate 3e was prepared from intermediate 3d (103 mg, 0.28 mmol) according to the procedure of step 3 in Example 1 to give a crude product (75 mg), which was directly used in the next step.

LCMS: [Ms+H]⁺ = 267.5.

### Step 6: Preparation of compound 3

Compound 3 was prepared from intermediate 1e (88 mg, 0.28 mmol) and intermediate 3e (75 mg, 0.28 mmol) according to the procedure of step 6 in Example 1, with a yield of 48.6%.

LCMS: [Ms+H]⁺ = 560.4.

¹H NMR (400 MHz, CDCl₃) *δ* 8.35 (s, 1H), 8.09 - 7.82 (m, 1H), 7.77 - 7.57 (m, 2H), 7.52 (d, J = 7.0 Hz, 1H), 7.46 - 7.30 (m, 2H), 4.07 - 3.70 (m, 2H), 3.49 - 3.27 (m, 3H), 3.24 - 2.81 (m, 7H), 2.81 - 2.25 (m, 3H), 1.78 - 1.46 (m, 4H), 1.45 - 1.27 (m, 8H), 1.16 (t, J = 6.9 Hz, 7H), 1.09 - 0.78 (m, 3H).

### Example 4: Preparation of Compound 4

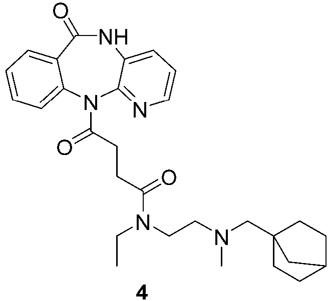

### Synthetic route of compound 4:

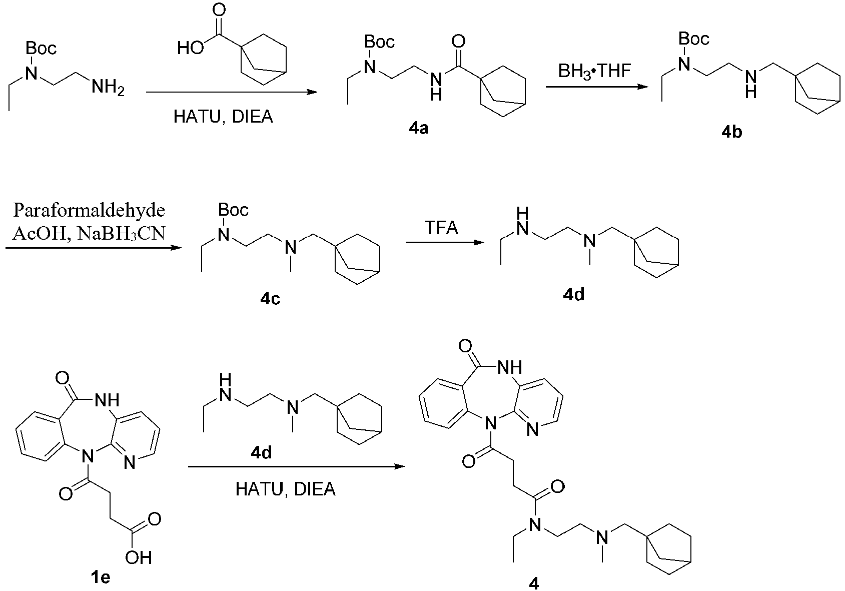

### Step 1: Preparation of intermediate 4a

*tert*-Butyl (2-aminoethyl)(ethyl)carbamate (403 mg, 2.14 mmol) was dissolved in N,N-dimethylformamide (5 mL), and then bicyclo[2.2.1]heptane-1-carboxylic acid (250 mg, 1.78 mmol), *N*,*N*,*N'*,*N'*-tetramethyl-*O*-(7-azabenzotriazol-1-yl)uronium hexafluorophospate (848 mg, 2.23 mmol), and N,N-diisopropylethylamine (1.15 mg, 8.92 mmol) were added. The resulting system was reacted at room temperature for 16 h under nitrogen atmosphere.

Water (10 mL) was added to quench the reaction. The aqueous phase was extracted three times with ethyl acetate (20 mL). The organic phases were combined, washed twice with brine (50 mL), dried over anhydrous sodium sulfate, filtered, and concentrated to dryness by rotary evaporation.

The resulting residue was purified by silica gel column chromatography (eluent: dichloromethane/methanol = 30/1) to give intermediate 4a (550 mg, yield: 99.3%).

LCMS: [Ms+H]⁺ = 311.2.

### Step 2: Preparation of intermediate 4b

Intermediate 4a (400 mg, 1.29 mmol) was dissolved in tetrahydrofuran (15 mL), and a 1 N borane-tetrahydrofuran complex (6.44 mL, 6.44 mmol) was added at 0 °C. The mixture was reacted at 70 °C for 2 h under nitrogen atmosphere.

Methanol (5 mL) was slowly added dropwise to quench the reaction, and then the mixture was stirred at 70 °C for another 16 h. The reaction solution was concentrated to dryness by rotary evaporation, and the residue was azeotroped twice with methanol (30 mL). The resulting crude product was intermediate 4b, which was directly used in the next step without further purification.

LCMS: [Ms+H]⁺ = 297.2.

### Step 3: Preparation of intermediate 4c

Intermediate 4c was prepared from intermediate 4b (380 mg, 1.28 mmol) and paraformaldehyde (385 mg, 12.82 mmol) according to the procedure of step 2 in Example 1 to give a colorless oil (560 mg).

LCMS: [Ms+H]⁺ = 311.2.

### Step 4: Preparation of intermediate 4d

Intermediate 4d was prepared from intermediate 4c (280 mg, 0.90 mmol) according to the procedure of step 3 in Example 1. The crude product was directly used in the next step without further purification.

LCMS: [Ms+H]⁺ = 211.2.

### Step 5: Preparation of compound 4

Compound 4 was prepared from intermediate 1e (252 mg, 0.81 mmol) and intermediate 4d (189 mg, 0.90 mmol) according to the procedure of step 6 in Example 1, with a yield of 16.6%.

LCMS: [Ms+H]⁺ = 504.3.

¹H NMR (400 MHz, CDCl₃) *δ* 8.35 (s, 1H), 7.96 (s, 1H), 7.74 - 7.49 (m, 3H), 7.42 (s, 1H), 7.31 (dd, J = 7.7, 4.7 Hz, 1H), 3.56 - 3.22 (m, 4H), 2.98 (brs, 1H), 2.86 - 2.71 (m, 1H), 2.69 - 2.39 (m, 6H), 2.30 (s, 2H), 2.15 (s, 1H), 1.57 (t, J = 12.0 Hz, 2H), 1.51 - 1.33 (m, 2H), 1.36 - 1.11 (m, 9H), 1.05 (t, J = 7.1 Hz, 1H).

### Example 5: Preparation of Compound 5

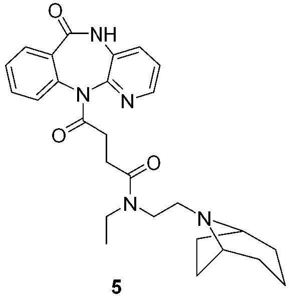

### Synthetic route of compound 5:

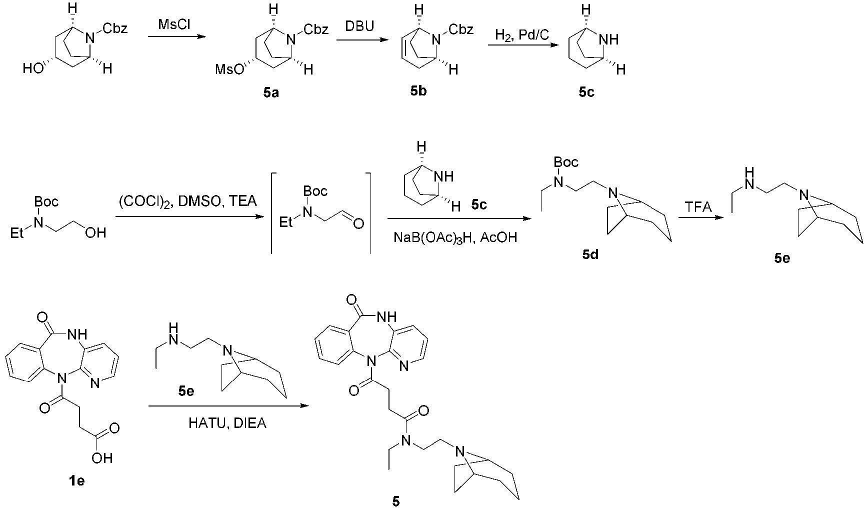

### Step 1: Preparation of intermediate 5a

Benzyl (1R,3r,5S)-3-hydroxy-8-azabicyclo[3.2.1]octane-8-carboxylate (2 g, 7.66 mmol) was dissolved in pyridine (15 mL), and then methanesulfonyl chloride (2.644 mg, 23.0 mmol) was added at 0 °C. The resulting system was reacted at room temperature for 3 h under nitrogen atmosphere.

The reaction solution was concentrated to dryness by rotary evaporation to give crude intermediate 5a (2.6 g), which was directly used in the next step without further purification.

LCMS: [Ms+H]⁺ =340.0.

### Step 2: Preparation of intermediate 5b

Intermediate 5a (2.6 g, 7.66 mmol) was dissolved in extra dry toluene (15 mL), and then 1,8-diazabicyclo[5.4.0]undec-7-ene (2.389 mg, 15.32 mmol) was added. The resulting system was reacted at 100 °C overnight under nitrogen atmosphere.

The reaction solution was concentrated to dryness by rotary evaporation. The resulting residue was purified by silica gel column chromatography (eluent: petroleum ether/ethyl acetate = 10/1) to give intermediate 5b (260 mg, yield: 13.95%).

LCMS: [Ms+H]⁺ =244.1.

### Step 3: Preparation of intermediate 5c

Intermediate 5b (260 mg, 1.07 mmol) was dissolved in methanol (15 mL), and then palladium on carbon (130 mg, 1.22 mmol) was added. The resulting system was reacted at room temperature overnight under hydrogen atmosphere.

The reaction solution was filtered through celite, and the resulting filtrate was concentrated to dryness by rotary evaporation to give crude intermediate 5c (139 mg), which was directly used in the next step without further purification.

LCMS: [Ms+H]⁺ =112.3.

### Step 4: Preparation of intermediate 5d

Intermediate 5d was prepared from tert-butyl ethyl(2-hydroxyethyl)carbamate (579 mg, 3.06 mmol) and intermediate 5c (170 mg, 1.53 mmol) according to the procedure of step 1 in Example 2, with a yield of 50.72%.

LCMS: [Ms+H]⁺ = 283.2.

### Step 5: Preparation of intermediate 5e

Intermediate 5e was prepared from intermediate 5d (100 mg, 0.355 mmol) according to the procedure of step 3 in Example 1 to give a crude product (65 mg), which was directly used in the next step.

LCMS: [Ms+H]⁺ = 183.2.

### Step 6: Preparation of compound 5

Compound 5 was prepared from intermediate 1e (64.6 mg, 0.355 mmol) and intermediate 5e (110 mg, 0.355 mmol) according to the procedure of step 6 in Example 1, with a yield of 17.92%.

LCMS: [Ms+H]⁺ = 476.2.

¹H NMR (400 MHz, CDCl₃) *δ* 8.96 (s, 1H), 8.36 (dd, J = 4.7, 1.5 Hz, 1H), 7.94 (d, J = 7.2 Hz, 1H), 7.63 (d, J = 7.0 Hz, 2H), 7.54 (d, J = 7.8 Hz, 1H), 7.48 - 7.29 (m, 2H), 4.04 - 3.87 (m, 2H), 3.75 (s, 1H), 3.55 - 3.35 (m, 3H), 3.17 - 2.84 (m, 4H), 2.46 (t, J = 12.8 Hz, 2H), 2.25 - 2.11 (m, 2H), 1.82 (s, 3H), 1.65 (d, J = 10.4 Hz, 2H), 1.49 (s, 2H), 1.42 - 1.32 (m, 1H), 1.20 (t, J = 6.9 Hz, 3H).

### Example 6: Preparation of Compound 6

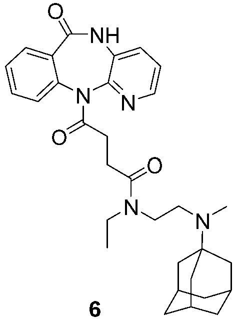

### Synthetic route of compound 6:

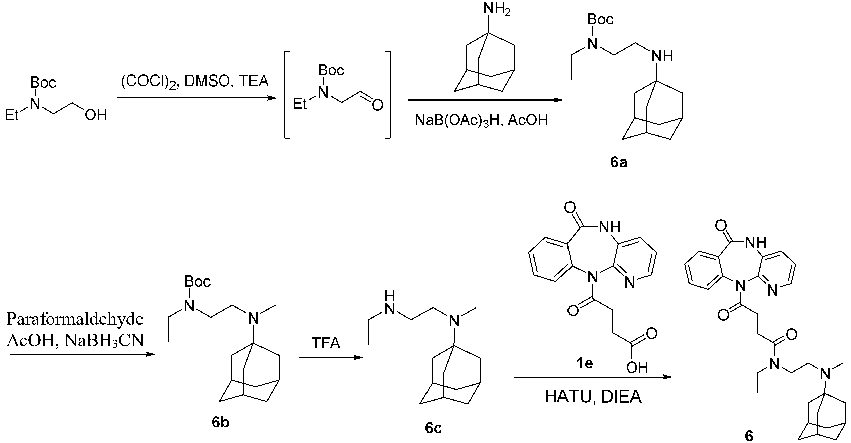

### Step 1: Preparation of intermediate 6a

Intermediate 6a was prepared from tert-butyl ethyl(2-hydroxyethyl)carbamate (450 mg, 2.38 mmol) and amantadine (300 mg, 1.98 mmol) according to the procedure of step 1 in Example 2, with a yield of 51.5%.

LCMS: [Ms+H]⁺ = 323.2.

### Step 2: Preparation of intermediate 6b

Intermediate 6b was prepared from intermediate 6a (330 mg, 1.02 mmol) and paraformaldehyde (307 mg, 10.23 mmol) according to the procedure of step 2 in Example 1 to give a colorless oil (300 mg).

LCMS: [Ms+H]⁺ = 337.3.

### Step 3: Preparation of intermediate 6c

Intermediate 6c was prepared from intermediate 6b (150 mg, 0.44 mmol) according to the procedure of step 3 in Example 1 to give a crude product (105 mg), which was directly used in the next step.

LCMS: [Ms+H]⁺ = 237.1.

### Step 4: Preparation of compound 6

Compound 6 was prepared from intermediate 1e (138 mg, 0.44 mmol) and intermediate 6c (105 mg, 0.44 mmol) according to the procedure of step 6 in Example 1, with a yield of 11.4%.

LCMS: [Ms+H]+ = 530.4.

¹H NMR (400 MHz, CDCl₃) *δ* 8.33 (d, J = 3.8 Hz, 1H), 7.91 (d, J = 8.5 Hz, 1H), 7.75 (d, J = 7.3 Hz, 1H), 7.68 - 7.57 (m, 1H), 7.54 (d, J = 7.8 Hz, 1H), 7.49 - 7.36 (m, 1H), 7.36 - 7.28 (m, 1H), 3.99 (s, 1H), 3.74 (s, 1H), 3.45 (d, J = 9.1 Hz, 2H), 3.11 (s, 1H), 2.98 - 2.55 (m, 6H), 2.53 - 2.34 (m, 2H), 2.19 (s, 3H), 2.07 - 1.79 (m, 10H), 1.20 (t, J = 7.1 Hz, 3H), 0.97 - 0.73 (m, 2H).

### Example 7: Preparation of Compound 7

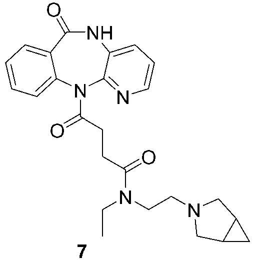

### Synthetic route of compound 7:

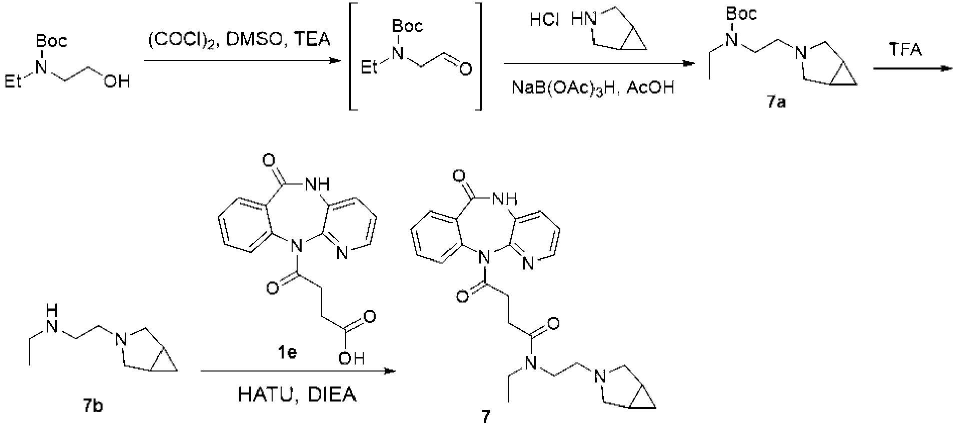

### Step 1: Preparation of intermediate 7a

Intermediate 7a was prepared from *tert*-butyl ethyl(2-hydroxyethyl)carbamate (500 mg, 2.65 mmol) and 3-azabicyclo[3.1.0]hexane (132.5 mg, 1.1 mmol) according to the procedure of step 1 in Example 2, with a yield of 98%.

LCMS: [Ms+H]⁺ = 255.1.

### Step 2: Preparation of intermediate 7b

Intermediate 7b was prepared from intermediate 7a (227 mg, 0.895 mmol) according to the procedure of step 3 in Example 1 to give a crude product (140 mg), which was directly used in the next step.

LCMS: [Ms+H]⁺ = 155.3.

### Step 3: Preparation of compound 7

Compound 7 was prepared from intermediate 1e (139 mg, 0.448 mmol) and intermediate 7b (107 mg, 0.895 mmol) according to the procedure of step 6 in Example 1, with a yield of 40.55%.

LCMS: [Ms+H]⁺ = 448.1.

¹H NMR (400 MHz, CDCl₃) *δ* 9.42 (s, 1H), 8.35 (s, 1H), 7.91 (d, J = 7.0 Hz, 1H), 7.62 (dd, J = 15.6, 8.1 Hz, 2H), 7.53 (d, J = 7.3 Hz, 1H), 7.42 (t, J = 6.8 Hz, 1H), 7.38 - 7.29 (m, 1H), 4.01 (d, J = 10.2 Hz, 1H), 3.79 (d, J = 10.4 Hz, 2H), 3.57 (s, 1H), 3.43 - 3.18 (m, 5H), 3.07 (s, 1H), 2.97 - 2.83 (m, 1H), 2.82 - 2.68 (m, 1H), 2.59 - 2.45 (m, 1H), 2.37 (d, J = 15.9 Hz, 1H), 1.69 (d, J = 3.9 Hz, 2H), 1.13 (t, J = 7.1 Hz, 3H), 0.88 (s, 1H), 0.74 (dd, J = 14.6, 7.5 Hz, 1H).

### Example 8: Preparation of Compound 8

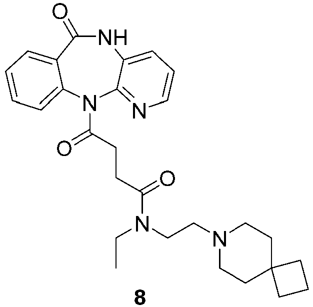

### Synthetic route of compound 8:

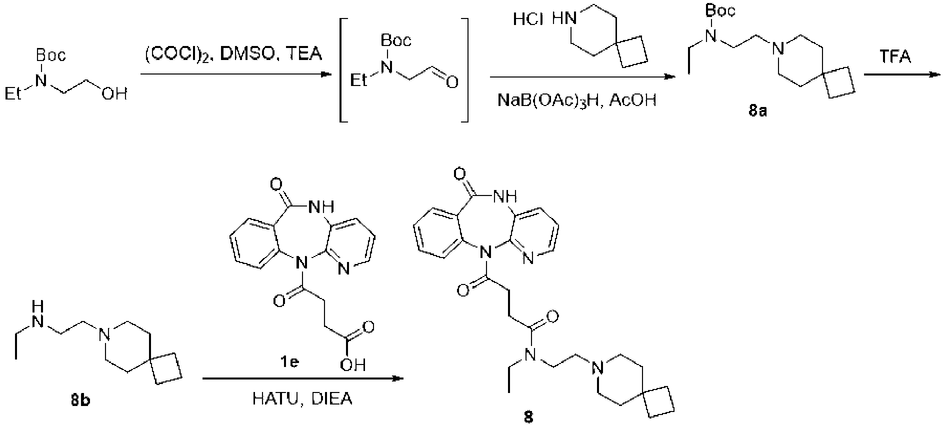

### Step 1: Preparation of intermediate 8a

Intermediate 8a was prepared from *tert*-butyl ethyl(2-hydroxyethyl)carbamate (351 mg, 1.86 mmol) and 7-azaspiro[3.5]nonane hydrochloride (200 mg, 1.24 mmol) according to the procedure of step 1 in Example 2, with a yield of 81.80%.

LCMS: [Ms+H]⁺ = 297.2.

### Step 2: Preparation of intermediate 8b

Intermediate 8b was prepared from intermediate 8a (150 mg, 0.51 mmol) according to the procedure of step 3 in Example 1 to give a crude product (99 mg), which was directly used in the next step.

LCMS: [Ms+H]⁺ = 197.2.

### Step 3: Preparation of compound 8

Compound 8 was prepared from intermediate 1e (105 mg, 0.34 mmol) and intermediate 8b (99 mg, 0.51 mmol) according to the procedure of step 6 in Example 1, with a yield of 12.20%.

LCMS: [Ms+H]⁺ = 490.2.

¹H NMR (400 MHz, CDCl₃) *δ* 8.34 (s, 1H), 7.97 (d, J = 7.2 Hz, 1H), 7.70 - 7.52 (m, 3H), 7.42 (s, 1H), 7.30 (dd, J = 7.9, 4.7 Hz, 1H), 3.49 - 3.26 (m, 4H), 2.97 (s, 1H), 2.81 - 2.72 (m, 1H), 2.68 - 2.58 (m, 1H), 2.52 - 2.29 (m, 7H), 1.88 - 1.81 (m, 2H), 1.73 - 1.67 (m, 4H), 1.60 - 1.53 (m, 4H), 1.18 - 1.00 (m, 3H).

### Example 9: Preparation of Compound 9

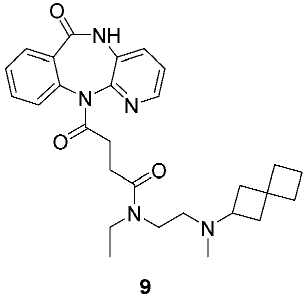

### Synthetic route of compound 9:

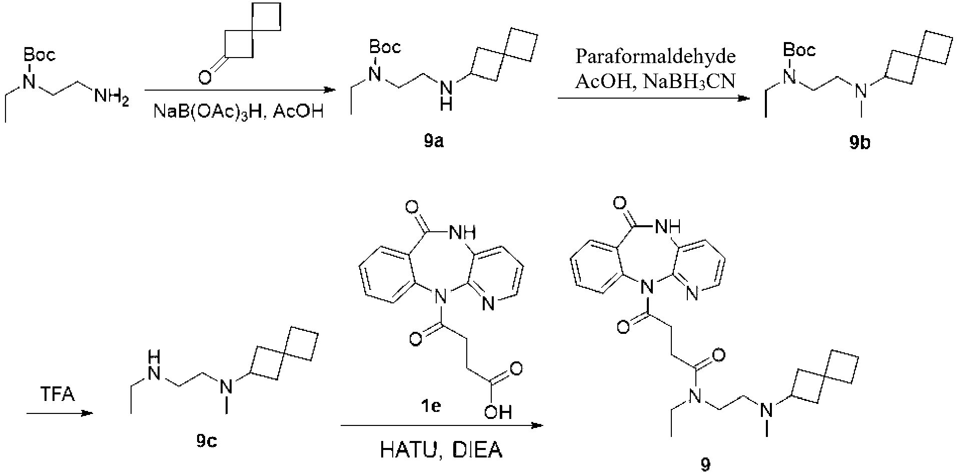

### Step 1: Preparation of intermediate 9a

Intermediate 9a was prepared from *tert*-butyl 2-aminoethyl(ethyl)carbamate (427 mg, 2.27 mmol) and spiro[3.3]heptan-2-one (250 mg, 2.27 mmol) according to the procedure of step 1 in Example 1, with a yield of 78.01%.

LCMS: [Ms+H]⁺ = 283.2.

### Step 2: Preparation of intermediate 9b

Intermediate 9b was prepared from intermediate 9a (500 mg, 1.77 mmol) and paraformaldehyde (532 mg, 17.70 mmol) according to the procedure of step 2 in Example 1, with a yield of 74.31%.

LCMS: [Ms+H]⁺ = 297.3.

### Step 3: Preparation of intermediate 9c

Intermediate 9c was prepared from intermediate 9b (100 mg, 0.34 mmol) according to the procedure of step 3 in Example 1 to give a crude product (67 mg), which was directly used in the next step.

LCMS: [Ms+H]⁺ = 197.2.

### Step 4: Preparation of compound 9

Compound 9 was prepared from intermediate 1e (106 mg, 0.34 mmol) and intermediate 9c (67 mg, 0.34 mmol) according to the procedure of step 6 in Example 1, with a yield of 33.32%.

LCMS: [Ms+H]⁺ = 490.2.

¹H NMR (400 MHz, CDCl₃) *δ* 8.36 (d, J = 3.7 Hz, 1H), 7.92 (s, 1H), 7.65 (dt, J = 14.1, 7.0 Hz, 2H), 7.52 (d, J = 7.7 Hz, 1H), 7.44 (s, 1H), 7.37 (dd, J = 7.9, 4.8 Hz, 1H), 4.67 (s, 3H), 3.88 (s, 1H), 3.48 - 3.31 (m, 3H), 2.94 - 2.69 (m, 5H), 2.46 - 2.16 (m, 5H), 2.05 - 1.88 (m, 4H), 1.84 (d, J = 7.4 Hz, 3H), 1.16 (t, J = 6.8 Hz, 3H).

### Example 10: Preparation of Compound 10

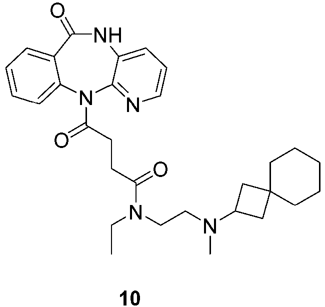

### Synthetic route of compound 10:

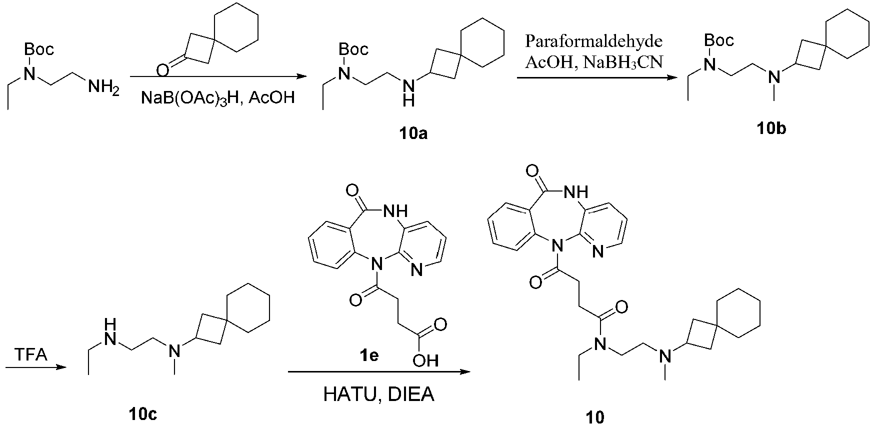

### Step 1: Preparation of intermediate 10a

Intermediate 10a was prepared from *tert*-butyl 2-aminoethyl(ethyl)carbamate (341 mg, 1.81 mmol) and spiro[3.5]nonan-2-one (250 mg, 1.81 mmol) according to the procedure of step 1 in Example 1, with a yield of 71.22%.

LCMS: [Ms+H]⁺ = 311.3.

### Step 2: Preparation of intermediate 10b

Intermediate 10b was prepared from intermediate 10a (400 mg, 1.29 mmol) and paraformaldehyde (387 mg, 12.88 mmol) according to the procedure of step 2 in Example 1, with a yield of 71.76%.

LCMS: [Ms+H]⁺ = 325.3.

### Step 3: Preparation of intermediate 10c

Intermediate 10c was prepared from intermediate 10b (150 mg, 0.46 mmol) according to the procedure of step 3 in Example 1 to give a crude product (104 mg), which was directly used in the next step.

LCMS: [Ms+H]⁺ = 225.2.

### Step 4: Preparation of compound 10

Compound 10 was prepared from intermediate 1e (144 mg, 0.46 mmol) and intermediate 10c (104 mg, 0.46 mmol) according to the procedure of step 6 in Example 1, with a yield of 26.61%.

LCMS: [Ms+H]⁺ = 518.3.

¹H NMR (400 MHz, CDCl₃) *δ* 8.97 - 8.48 (m, 1H), 8.35 (s, 1H), 7.96 (s, 1H), 7.73 - 7.51 (m, 3H), 7.43 (s, 1H), 7.30 (dd, J = 7.8, 4.7 Hz, 1H), 3.58 - 3.24 (m, 4H), 2.97 (s, 1H), 2.87 - 2.67 (m, 2H), 2.66 - 2.57 (m, 1H), 2.54 - 2.28 (m, 3H), 2.26 - 2.09 (m, 2H), 1.98 - 1.87 (m, 2H), 1.49 - 1.30 (m, 11H), 1.21 - 1.01 (m, 3H).

### Example 11: Preparation of Compound 11

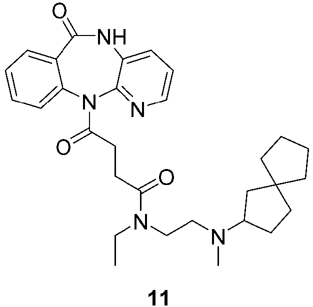

### Synthetic route of compound 11:

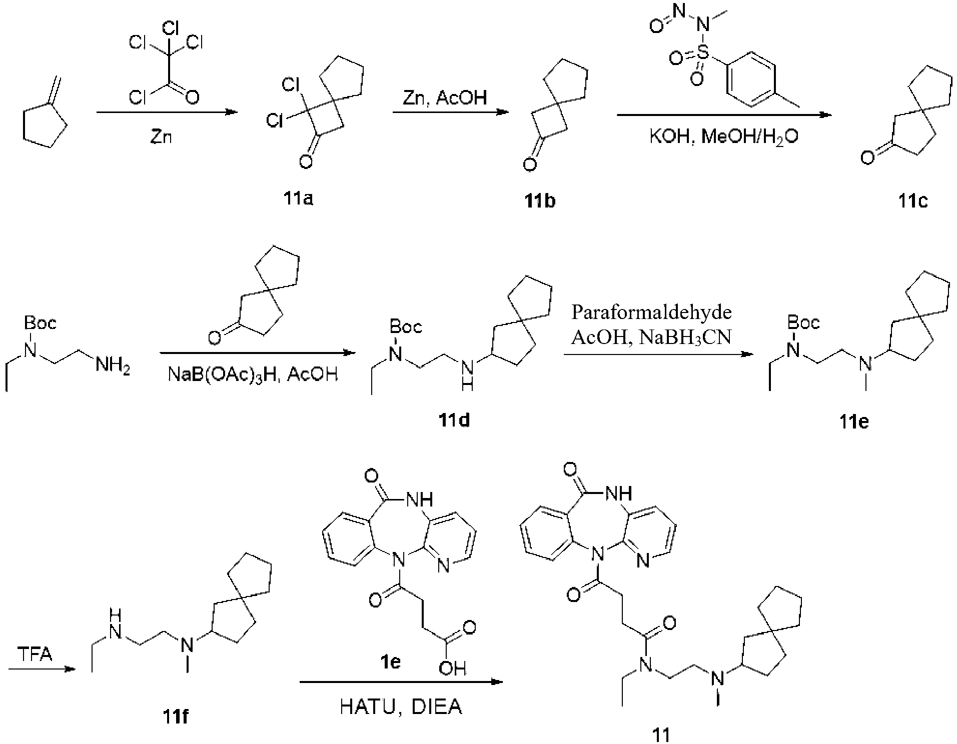

### Step 1: Preparation of intermediate 11a

Methylenecyclopentane (1.50 g, 18.26 mmol) was dissolved in diethyl ether (15 mL), and zinc powder (2.39 g, 36.52 mmol) was added at 0 °C. Subsequently, trichloroacetyl chloride (4.98 g, 27.39 mmol) was slowly added dropwise. The resulting system was stirred at 40 °C for 1.5 h under nitrogen atmosphere.

The reaction solution was filtered through celite and washed twice with diethyl ether (30 mL). The organic phases were combined, washed sequentially with water (100 mL), a saturated aqueous sodium bicarbonate solution (100 mL), and brine (100 mL), dried over anhydrous sodium sulfate, and concentrated. The residue was concentrated to give crude intermediate 11a (3.00 g) as a colorless oil, which was directly used in the next step.

TLC: Rf= 0.7 (PE/EA = 10/1).

### Step 2: Preparation of intermediate 11b

Zinc powder (25.40 g, 388.47 mmol) was dissolved in acetic acid (50 mL), and a solution of intermediate 11a (3.00 g, 15.54 mmol) in acetic acid (10 mL) was slowly added dropwise at room temperature. The resulting mixture was reacted at 60 °C for 2 h under nitrogen atmosphere.

The reaction solution was filtered through celite and washed twice with *n*-pentane (100 mL). The organic phases were combined, and water (100 mL) was added. The resulting mixture was then extracted with n-pentane (100 mL). The organic phase was washed with a 1 N aqueous sodium hydroxide solution (50 mL × 2) and brine (100 mL), dried over anhydrous sodium sulfate, and concentrated.

The resulting residue was purified by a normal phase column (eluent: petroleum ether/ethyl acetate = 20/1) to give intermediate 11b (300 mg, yield: 15.55%).

¹H NMR (400 MHz, CDCl₃) *δ* 2.91 (s, 4H), 1.83 - 1.65 (m, 8H).

### Step 3: Preparation of intermediate 11c

Potassium hydroxide (1.08 g, 19.33 mmol) was dissolved in methanol (5 mL) and water (1 mL), and a solution of intermediate 11b (200 mg, 1.61 mmol) in methanol (5 mL) was slowly added dropwise at 0 °C. After the dropwise addition, N-methyl-N-nitroso-p-toluenesulfonamide (449 mg, 2.09 mmol) was added. The resulting mixture was reacted at room temperature for 0.5 h under nitrogen atmosphere.

Acetic acid (1 mL) and water (10 mL) were added to the reaction solution, and the mixture was then extracted three times with n-pentane (30 mL). The organic phases were combined, washed once with brine (100 mL), dried over anhydrous sodium sulfate, and concentrated to dryness by rotary evaporation.

The resulting residue was purified by a normal phase column (eluent: petroleum ether/ethyl acetate = 20/1) to give intermediate 11c (130 mg, yield: 58.40%).

¹H NMR (400 MHz, CDCl₃) *δ* 1.78 - 1.47 (m, 4H), 1.38 - 1.00 (m, 5H), 0.92 - 0.81 (m, 5H).

### Step 4: Preparation of intermediate 11d

Intermediate 11d was prepared from *tert*-butyl 2-aminoethyl(ethyl)carbamate (180 mg, 0.96 mmol) and intermediate 11c (132 mg, 0.96 mmol) according to the procedure of step 1 in Example 1, with a yield of 67.38%.

LCMS: [Ms+H]⁺ = 311.2.

### Step 5: Preparation of intermediate 11e

Intermediate 11e was prepared from intermediate 11d (170 mg, 0.55 mmol) and paraformaldehyde (164 mg, 5.48 mmol) according to the procedure of step 2 in Example 1, with a yield of 88.50%.

LCMS: [Ms+H]⁺ = 325.2.

### Step 6: Preparation of intermediate 11f

Intermediate 11f was prepared from intermediate 11e (100 mg, 0.31 mmol) according to the procedure of step 3 in Example 1 to give a crude product (69 mg), which was directly used in the next step.

LCMS: [Ms+H]⁺ = 225.2.

### Step 7: Preparation of compound 11

Compound 11 was prepared from intermediate 1e (105 mg, 0.34 mmol) and intermediate 11f (69 mg, 0.34 mmol) according to the procedure of step 6 in Example 1, with a yield of 28.88%.

LCMS: [Ms+H]⁺ = 518.2.

¹H NMR (400 MHz, CDCl₃) *δ* 9.28 (s, 1H), 8.34 (s, 1H), 7.97 (d, J = 7.7 Hz, 1H), 7.73 - 7.52 (m, 3H), 7.42 (s, 1H), 7.30 (dd, J = 7.8, 4.7 Hz, 1H), 3.55 - 3.25 (m, 4H), 3.00 (s, 1H), 2.90 - 2.71 (m, 2H), 2.69 - 2.39 (m, 4H), 2.34 - 2.19 (m, 3H), 1.84 (d, J = 10.3 Hz, 1H), 1.75 - 1.65 (m, 1H), 1.60 - 1.34 (m, 12H), 1.20 - 1.03 (m, 3H).

### Example 12: Preparation of Compound 12

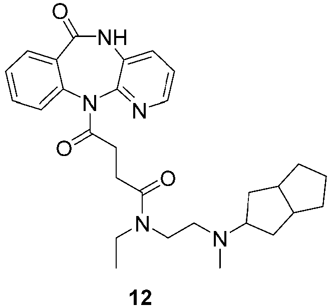

### Synthetic route of compound 12:

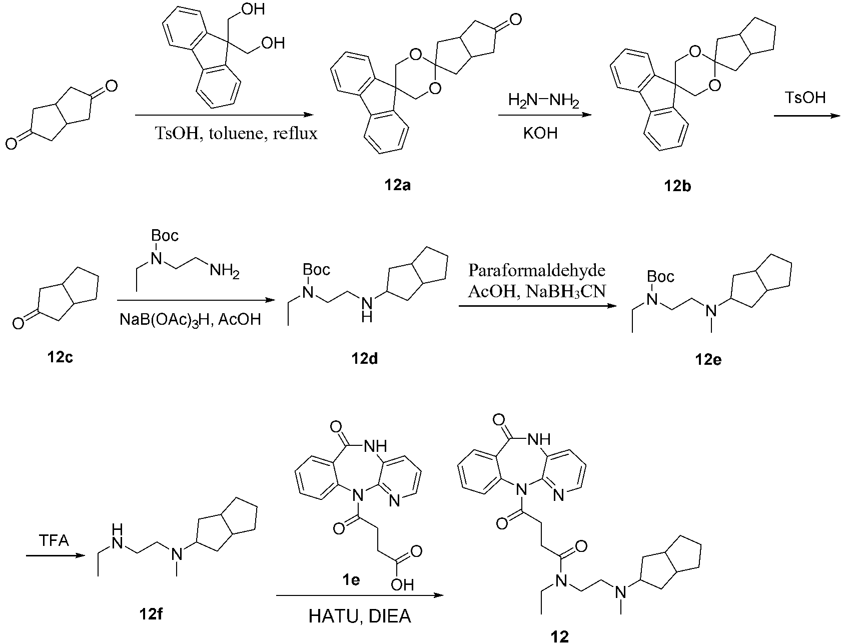

### Step 1: Preparation of intermediate 12a

Bicyclo[3.3.0]octane-3,7-dione (2.00 g, 14.48 mmol) was dissolved in toluene (40 mL), and then 9H-fluorene-9,9-dimethanol (3.28 g, 14.48 mmol) and p-toluenesulfonic acid (249 mg, 1.45 mmol) were added. The resulting system was stirred at 110 °C for 4 h under nitrogen atmosphere.

The reaction solution was directly concentrated to dryness by rotary evaporation. The resulting residue was purified by silica gel column chromatography (eluent: petroleum ether/ethyl acetate = 8/1) to give intermediate 12a (2.50 g, yield: 49.85%).

TLC: Rf = 0.5 (PE/EA = 4/1).

### Step 2: Preparation of intermediate 12b

Intermediate 12a (2.50 g, 7.22 mmol) was dissolved in diethylene glycol (30 mL), and hydrazine hydrate (4.34 g, 84.60 mmol) and potassium hydroxide (2.02 g, 36.08 mmol) were added. The resulting mixture was reacted at 160 °C for 1 h under nitrogen atmosphere, and then reacted at 205 °C for 2 h.

Water (50 mL) was added to quench the reaction. The mixture was then extracted three times with methyl *tert*-butyl ether (50 mL). The organic layers were combined, washed once with brine (100 mL), dried over anhydrous sodium sulfate, and concentrated to dryness by rotary evaporation.

The resulting residue was purified by a normal phase column (eluent: petroleum ether/ethyl acetate = 20/1) to give intermediate 12b (2.25 g, yield: 93.79%).

TLC: Rf = 0.8 (PE:EA = 10/1).

### Step 3: Preparation of intermediate 12c

Intermediate 12b (450 mg, 1.352 mmol) was dissolved in tetrahydrofuran (10 mL), and a 3 N aqueous hydrochloric acid solution (4.51 mL, 13.54 mmol) was added. The resulting mixture was reacted at 70 °C for 2 h under nitrogen atmosphere.

The reaction system was cooled to room temperature, adjusted to pH = 8 with a saturated aqueous sodium bicarbonate solution, and then extracted three times with ethyl acetate (30 mL). The organic layers were combined, washed once with brine (100 mL), dried over anhydrous sodium sulfate, and concentrated to dryness by rotary evaporation to give crude intermediate 12c (158 mg), which was directly used in the next step.

TLC: Rf = 0.7 (PE:EA = 10/1).

### Step 4: Preparation of intermediate 12d

Intermediate 12d was prepared from *tert*-butyl 2-aminoethyl(ethyl)carbamate (300 mg, 1.59 mmol) and intermediate 12c (237 mg, 1.91 mmol) according to the procedure of step 1 in Example 1, with a yield of 52.29%.

LCMS: [Ms+H]⁺ = 297.2.

### Step 5: Preparation of intermediate 12e

Intermediate 12e was prepared from intermediate 12d (250 mg, 0.84 mmol) and paraformaldehyde (253 mg, 8.43 mmol) according to the procedure of step 2 in Example 1, with a yield of 99.30%.

LCMS: [Ms+H]⁺ = 311.2.

### Step 6: Preparation of intermediate 12f

Intermediate 12f was prepared from intermediate 12e (100 mg, 0.32 mmol) according to the procedure of step 3 in Example 1 to give a crude product (67 mg), which was directly used in the next step.

LCMS: [Ms+H]⁺ = 211.2.

### Step 7: Preparation of compound 12

Compound 12 was prepared from intermediate 1e (99 mg, 0.32 mmol) and intermediate 12f (67 mg, 0.32 mmol) according to the procedure of step 6 in Example 1, with a yield of 38.41%.

LCMS: [Ms+H]⁺ = 504.3.

¹H NMR (400 MHz, CDCl₃) *δ* 8.35 (s, 1H), 7.97 (d, J = 7.9 Hz, 1H), 7.70 - 7.51 (m, 3H), 7.42 (s, 1H), 7.30 (dd, J = 7.9, 4.7 Hz, 1H), 3.53 - 3.26 (m, 4H), 2.97 (s, 1H), 2.81 - 2.45 (m, 6H), 2.36 (s, 3H), 2.15 - 2.00 (m, 2H), 1.81 - 1.51 (m, 6H), 1.39 (d, J = 5.0 Hz, 2H), 1.20 - 0.93 (m, 5H).

### Example 13: Preparation of Compound 13

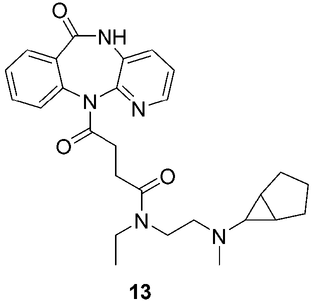

### Synthetic route of compound 13:

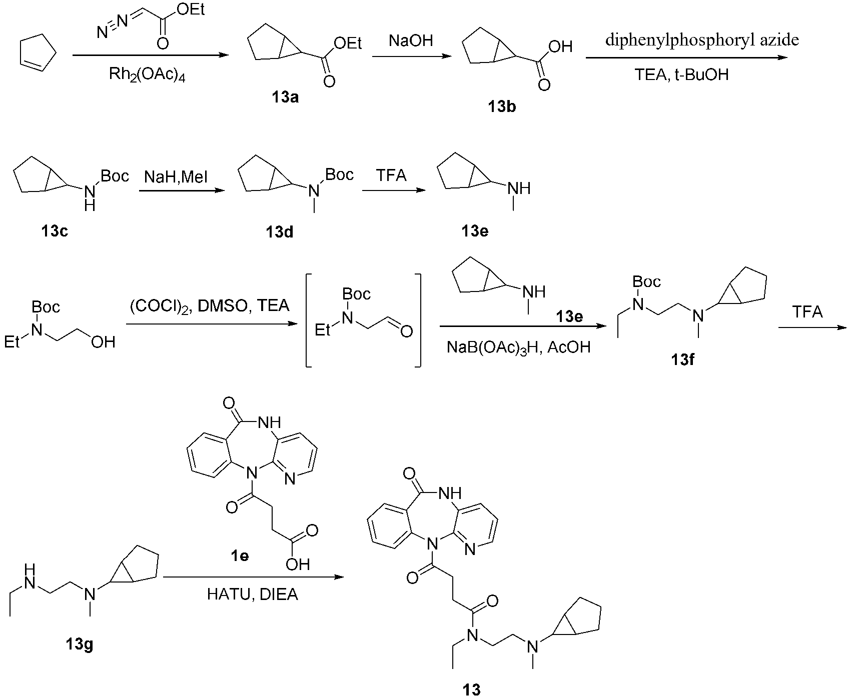

### Step 1: Preparation of intermediate 13a

Cyclopentene (3.07 g, 45 mmol) and rhodium(II) acetate dimer (59.7 mg, 0.003 mmol) were dissolved in diethyl ether (60 mL), and then a solution of ethyl diazoacetate (8.85 g, 77.6 mmol) in diethyl ether (15 mL) was slowly added dropwise over 5 h.

The reaction solution was concentrated to dryness by rotary evaporation. The resulting residue was purified by silica gel column chromatography to give intermediate 13a (1.2 g, yield: 17.27%).

¹H NMR (400 MHz, CDCl₃) *δ* 4.13 - 4.06 (m, 2H), 1.88 - 1.51 (m, 6H), 1.41 - 1.20 (m, 5H), 1.16 - 0.85 (m, 1H).

### Step 2: Preparation of intermediate 13b

Intermediate 13a (600 mg, 3.9 mmol) was dissolved in methanol (20 mL), and a sodium hydroxide solution (4 mol/L, 10 mL) was added. The resulting system was reacted at room temperature overnight under nitrogen atmosphere.

The reaction solution was concentrated to dryness by rotary evaporation. The resulting residue was dissolved in water (50 mL) and extracted with diethyl ether (50 mL). The aqueous phase was adjusted to pH = 2 with 2 mol/L hydrochloric acid and extracted three times with diethyl ether. The organic phases were combined, dried over anhydrous sodium sulfate, and filtered. The filtrate was concentrated to dryness by rotary evaporation to give crude intermediate 13b (333 mg), which was directly used in the next step without further purification.

LCMS: [Ms-H] = 125.1.

### Step 3: Preparation of intermediate 13c

Intermediate 13b (360 mg, 2.86 mmol) was dissolved in *tert*-butanol (15 mL), and triethylamine (317 mg, 3.14 mmol) and diphenylphosphoryl azide (864 mg, 3.14 mmol) were added. The resulting mixture was reacted at 80 °C for 48 h under nitrogen atmosphere.

The reaction solution was concentrated to dryness by rotary evaporation. Water (50 mL) was added to the resulting residue, and the mixture was extracted three times with diethyl ether. The organic phases were combined, washed sequentially with a saturated sodium bicarbonate solution and a saturated sodium chloride solution, dried over anhydrous sodium sulfate, and filtered. The filtrate was concentrated to dryness by rotary evaporation.

The resulting residue was purified by silica gel column chromatography (eluent: petroleum ether/ethyl acetate = 10%) to give intermediate 13c (140 mg, yield: 24.87%).

LCMS: [Ms+H-56] = 142.2.

### Step 4: Preparation of intermediate 13d

Intermediate 13c (100 mg, 0.5 mmol) was dissolved in *N,N-*dimethylformamide (5 mL), and sodium hydride (100 mg, 2.5 mmol) was added at 0 °C. The resulting mixture was stirred at room temperature for 1 h, and then iodomethane (355 mg, 2.5 mmol) was added at 0 °C. The resulting system was reacted at room temperature for another 1 h under nitrogen atmosphere.

A saturated ammonium chloride solution (10 mL) was added to the reaction solution to quench the reaction, and the mixture was extracted with ethyl acetate (200 mL). The organic phase was washed twice with a saturated sodium chloride solution (50 mL), dried over anhydrous sodium sulfate, and concentrated to dryness by rotary evaporation.

The resulting residue was purified by silica gel column chromatography (eluent: petroleum ether/ethyl acetate = 10:1) to give intermediate 13d (68 mg, yield: 63.49%).

LCMS: [Ms+H-56] = 156.2.

### Step 5: Preparation of intermediate 13e

Intermediate 13d (88 mg, 0.417 mmol) was dissolved in dichloromethane (2 mL), and trifluoroacetic acid (1 mL) was added dropwise at 0 °C. The mixture was reacted at room temperature for 1 h.

The reaction solution was concentrated to dryness by rotary evaporation, and the resulting residue was azeotroped three times with dichloromethane (20 mL). The mixture was concentrated to dryness by rotary evaporation without further purification to give intermediate 13e (50 mg), which was directly used in the next step.

LCMS: [Ms+H] = 112.3.

### Step 6: Preparation of intermediate 13f

Intermediate 13f was prepared from *tert*-butyl ethyl(2-hydroxyethyl)carbamate (158 mg, 0.834 mmol) and intermediate 13e (46 mg, 0.417 mmol) according to the procedure of step 1 in Example 2, with a yield of 98%.

LCMS: [Ms+H]⁺ = 283.3.

### Step 7: Preparation of intermediate 13g

Intermediate 13g was prepared from intermediate 13f (130 mg, 0.46 mmol) according to the procedure of step 3 in Example 1 to give a crude product (85 mg), which was directly used in the next step.

LCMS: [Ms+H]⁺ = 183.3.

### Step 8: Preparation of compound 13

Compound 13 was prepared from intermediate 1e (286 mg, 0.92 mmol) and intermediate 13g (84 mg, 0.46 mmol) according to the procedure of step 6 in Example 1, with a yield of 44.3%.

LCMS: [Ms+H]⁺ = 476.1.

¹H NMR (400 MHz, CDCl₃) *δ* 8.36 (s, 1H), 8.07 - 7.84 (m, 1H), 7.75 - 7.59 (m, 2H), 7.53 (d, J = 6.3 Hz, 1H), 7.43 (s, 1H), 7.36 (dd, J = 7.3, 4.8 Hz, 1H), 3.90 (s, 1H), 3.52 - 3.08 (m, 5H), 3.05 - 2.63 (m, 5H), 2.60 - 2.32 (m, 2H), 2.30 - 2.12 (m, 1H), 1.96 (s, 2H), 1.86 - 1.58 (m, 5H), 1.17 (t, J = 7.0 Hz, 3H), 1.09 - 0.95 (m, 1H).

### Example 14: Preparation of Compound 14

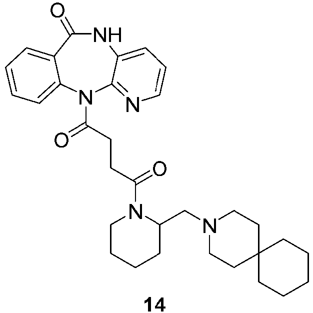

### Synthetic route of compound 14:

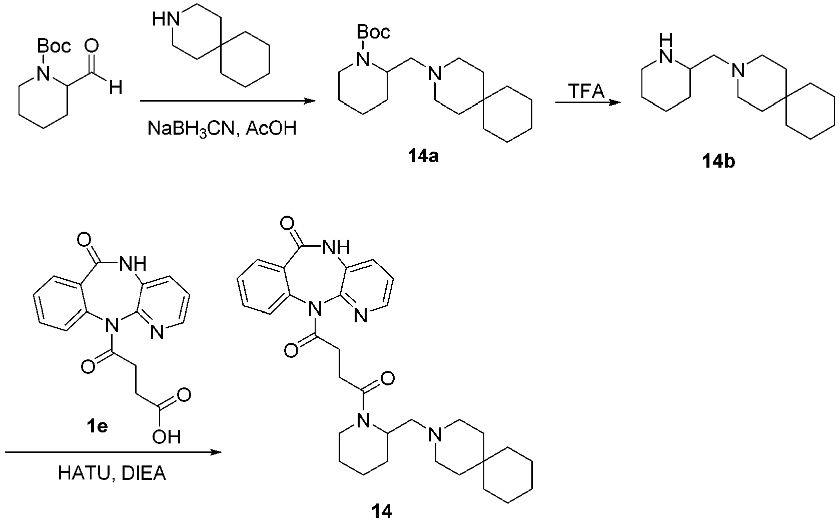

### Step 1: Preparation of intermediate 14a

*tert*-Butyl 2-formylpiperidine-1-carboxylate (213 mg, 1 mmol) was dissolved in methanol (10 mL), and 3-azaspiro[5.5]undecane (153 mg, 1 mmol), acetic acid (120 mg, 2 mmol), and sodium cyanoborohydride (189 mg, 3 mmol) were sequentially added. The mixture was reacted at room temperature for 4 h under nitrogen atmosphere.

A saturated aqueous sodium bicarbonate solution (20 mL) was added to quench the reaction. The aqueous phase was extracted three times with dichloromethane (100 mL). The organic phases were combined, dried over anhydrous sodium sulfate, filtered, and concentrated to dryness by rotary evaporation.

The resulting residue was purified by silica gel column chromatography (eluent: dichloromethane/methanol = 20/1) to give intermediate 14a (290 mg, yield: 82.84%).

LCMS: [Ms+H]⁺ = 351.3.

### Step 2: Preparation of intermediate 14b

Intermediate 14b was prepared from intermediate 14a (150 mg, 0.428 mmol) according to the procedure of step 3 in Example 1 to give a crude product (110 mg), which was directly used in the next step.

LCMS: [Ms+H]⁺ = 251.2.

### Step 3: Preparation of compound 14

Compound 14 was prepared from intermediate 1e (133 mg, 0.428 mmol) and intermediate 14b (107 mg, 0.428 mmol) according to the procedure of step 6 in Example 1, with a yield of 26.24%.

LCMS: [Ms+H]⁺ = 544.2.

¹H NMR (400 MHz, CDCl₃) *δ* 9.38 (d, J = 27.3 Hz, 1H), 8.34 (s, 1H), 7.97 (d, J = 7.4 Hz, 1H), 7.60 (d, J = 6.9 Hz, 3H), 7.42 (s, 1H), 7.29 (dd, J = 7.8, 4.7 Hz, 1H), 4.86 (s, 0.5H), 4.44 (d, J = 13.1 Hz, 0.5H), 4.05 (s, 0.5H), 3.71 (d, J = 13.1 Hz, 0.5H), 3.14 - 2.96 (m, 1H), 2.94 - 2.55 (m, 3H), 2.54 - 2.29 (m, 7H), 1.83 - 1.68 (m, 1H), 1.65 - 1.46 (m, 4H), 1.37 (s, 10H), 1.26 (s, 5H).

### Example 15: Preparation of Compound 15

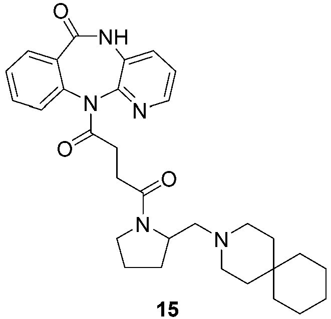

### Synthetic route of compound 15:

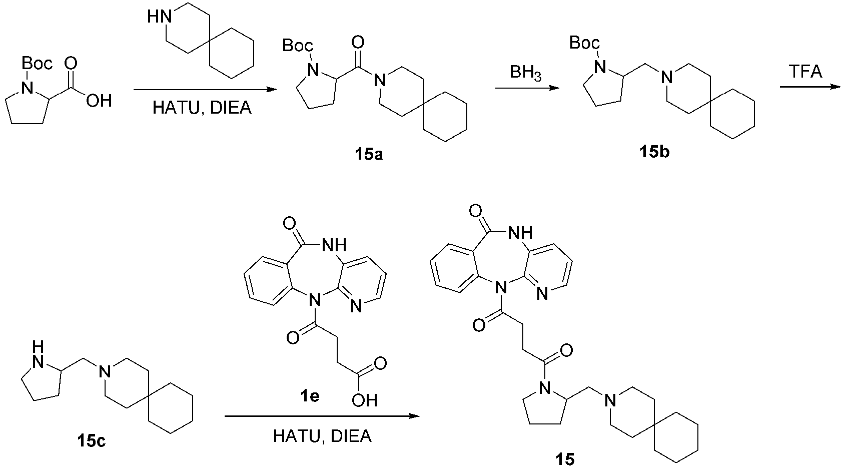

### Step 1: Preparation of intermediate 15a

1-(*tert*-Butoxycarbonyl)pyrrolidine-2-carboxylic acid (300 mg, 1.39 mmol) and 3-azaspiro[5.5]undecane (214 mg, 1.39 mmol) were dissolved in N,N-dimethylformamide (5 mL), and *N,N*-diisopropylethylamine (721 mg, 5.57 mmol) and *O*-(7-azabenzotriazol-1-yl)-*N*,*N*,*N'*,*N'*-tetramethyluronium hexafluorophosphate (636 mg, 3.19 mmol) were sequentially added. The mixture was reacted at room temperature for 16 h under nitrogen atmosphere.

The reaction system was diluted with water (50 mL) and extracted three times with dichloromethane (30 mL). The organic phases were combined, washed with brine (50 mL), dried over anhydrous sodium sulfate, and concentrated.

The resulting residue was purified by silica gel column chromatography (eluent: dichloromethane/methanol = 20/1) to give intermediate 15a (450 mg, yield: 92.12%).

LCMS: [Ms+H]⁺ = 351.2.

### Step 2: Preparation of intermediate 15b

Intermediate 15a (450 mg, 1.28 mmol) was dissolved in tetrahydrofuran (10 mL), and then a 1 N solution of borane in tetrahydrofuran (6.42 mL, 6.42 mmol) was added dropwise at room temperature. The resulting system was stirred at 70 °C for 2 h under nitrogen atmosphere.

Methanol (10 mL) was added to quench the reaction, and the mixture was stirred at 70 °C overnight. The reaction solution was directly concentrated to dryness by rotary evaporation and azeotroped twice with methanol (20 mL) to give crude intermediate 15b (300 mg), which was directly used in the next step without further purification.

LCMS: [Ms+H]⁺ =337.2.

### Step 3: Preparation of intermediate 15c

Intermediate 15c was prepared from intermediate 15b (100 mg, 0.30 mmol) according to the procedure of step 3 in Example 1 to give a crude product (70 mg), which was directly used in the next step.

LCMS: [Ms+H]⁺ = 237.2.

### Step 4: Preparation of compound 15

Compound 15 was prepared from intermediate 1e (92 mg, 0.30 mmol) and intermediate 15c (70 mg, 0.30 mmol) according to the procedure of step 6 in Example 1, with a yield of 65.03%.

LCMS: [Ms+H]⁺ = 530.2.

¹H NMR (400 MHz, CDCl₃) *δ* 8.35 (d, J = 3.3 Hz, 1H), 8.03 - 7.79 (m, 3H), 7.72 - 7.60 (m, 2H), 7.52 (d, J = 7.2 Hz, 1H), 7.41 - 7.35 (m, 1H), 4.52 - 4.26 (m, 1H), 3.85 - 3.38 (m, 4H), 3.20 - 2.57 (m, 6H), 2.41 (t, J = 11.7 Hz, 2H), 2.20 - 2.05 (m, 1H), 2.01 - 1.85 (m, 2H), 1.84 - 1.61 (m, 4H), 1.57 - 1.21 (m, 11H).

### Example 16: Assay for Inhibitory Activity of Compounds Against Human M2 and M3 Receptors

### Experimental principle

CHO cell lines stably expressing Human M2 and Human M3, respectively, were used. The effects of the compounds on the activity of the Human M2 and Human M3 receptors were determined using the FLIPR Calcium 6 Assay Kit. The effects of the test substances on Human M2 and Human M3 were studied according to changes in cell signal intensity, and the corresponding concentration-response curves were calculated.

Experimental reagents, consumables, and instruments are shown in Tables 1 and 2.

**Table 1. Sources of reagents and consumables**

| **Reagent and consumable** | **Manufacturer** | **Cat. No.** |
|---|---|---|
| F-12 | Hyclone | SH30026.01 |
| FLIPR CALCIUM 6 ASSAY KIT | Molecular Devices | R8191 |
| Fetal Bovine Serum (FBS) | AusGeneX | FBS500-S |
| 0.25% Trypsin-EDTA | Gibco | 25200-072 |
| Hygromycin B | Solarbio | H8080-1g |
| DMSO | Sigma | D4540 |
| HBSS | Gibco | 14025-092 |
| HEPES | Solarbio | No.H1095 |
| 96-well Dilution Plates-0.36-Sterilization | Biao Sen | P-0.6-BSA-96-S |
| 384-well Conical Bottom Polypropylene Plates | Corning | 264573 |
| Corning^{®} 384-well Flat Clear Bottom Black Polystyrene TC-treated Microplates | Corning | 3764 |

**Table 2. Instrument information**

| **Instrument** | **Manufacturer** | **Model** |
|---|---|---|
| Biosafety cabinet | ECSO | AC2-6S1-TC |
| Carbon dioxide incubator | ESCO (Singapore) | CCl-170B-8 |
| Inverted microscope | Olympus | CKX53 |
| Microplate low speed centrifuge | Xiangzhi | TD5B |
| FLIPR^{®} Penta | Molecular Devices (Shanghai) | 5.0 |

### Cell information

1. The Human M2-CHO and Human M3-CHO cell lines were cultured in an F-12 culture medium containing 10% fetal bovine serum (AusGeneX, FBS500-S) and 0.2 mg/mL Hygromycin B (Solarbio, H8080-1g) at a culture temperature of 37 °C with 5% carbon dioxide.
2. Cell passage: The old culture medium was removed in a biosafety cabinet, and the cells were washed once with PBS; 1 mL of 0.25% Trypsin-EDTA (Gibco, 25200-072) solution was then added; and the cells were incubated at 37 °C for about 2 min. When the cells were detached from the bottom of the dish, about 5 mL of complete culture medium pre-heated at 37 °C was added. The cell suspension was gently pipetted to separate the aggregated cells. The cell suspension was transferred to a sterile centrifuge tube and centrifuged at 1000 rpm for 5 min.
3. In order to maintain the physiological activity of the cells, the experimental cell confluence was controlled at about 80% as observed under an inverted microscope (Olympus, CKX53).

### Experimental procedures

1. Cell plating: Human M2-CHO and Human M3-CHO cells were digested, collected, resuspended, counted, and then seeded into a 384-well cell plate (Corning, 3764) at a density of 1.2 × 10⁴ cells/25 µL /well; the cell plate was then incubated in a 5% CO₂ incubator at 37 °C for about 16-20 h.
2. On day 2, an Assay Buffer (20 mM HEPES + 1× HBSS) was prepared according to the instructions of the FLIPR Calcium 6 Assay Kit (Molecular Devices, R8191); after 20× Component A was freeze-thawed to room temperature, it was diluted to 1× loading buffer with the Assay buffer, and placed at room temperature for later use.
3. The culture medium in the cell plate was removed, 35 µL of the 1× loading buffer described above was quickly added to each well, and the cell plate was centrifuged using a microplate low speed centrifuge and then incubated in the dark at 37 °C for 120 min.
4. The working solutions of a positive compound and the test compounds were each prepared in a 96-well dilution plate (Biao Sen, P-0.6-BSA-96-S); 5 µL of each solution was transferred to the corresponding cell wells; and the cell plate was incubated at 37 °C for 30 min in the dark.
5. An agonist working solution was prepared and transferred to a 384-well compound plate (Corning, 264573) at 20 µL /well.
6. The cell plate (Corning, 3764), the 384-well compound plate (Corning, 264573), and pipette tips were placed at corresponding positions of the FLIPR instrument; 10 µL of the agonist diluted in step 5 was added to each experimental well using the FLIPR^{®} Penta; and data were collected at the wavelength of 515-575 nm.
7. The signal values were plotted against the compound concentrations, and based on a non-linear regression method using the GraphPad Prism software, the curves were fit, and the IC₅₀ values were calculated.

### Data Analysis

1) $Z ′ factor = 1 - 3 \times \left({SD}_{Max}+{SD}_{Min}\right) / \left({AVG}_{Max}-{AVG}_{Min}\right)$
2) $CV Max = \left({SD}_{Max}/{AVG}_{Max}\right) \times 100 %$
3) $CV Min = \left({SD}_{Min}/{AVG}_{Min}\right) \times 100 %$
4) $Signal - to - noise ratio S / B = {AVG}_{Max} / {AVG}_{Min}$
5) The IC₅₀ values of the compounds were calculated using the GraphPad Prism non-linear fitting formula: $Y = Bottom + \left(Top-Bottom\right) / \left(1+{10}^{∧}\left(\left({LogIC}_{50}-X\right)\times HillSlope\right)\right)$
6) % inhibition rate formula: $% In h ibition = \left⌊\frac{{Ratio}_{cmpd} - {\overline{Ratio}}_{ve h icle}}{{\overline{Ratio}}_{positive} - {\overline{Ratio}}_{ve h icle}}\right⌋ \times 100$
   R̅a̅t̅i̅o̅*ₚₒₛᵢₜᵢᵥₑ*: mean value of positive control.
   R̅a̅t̅i̅o̅*_{vehicle}*: mean value of negative control (0.1% DMSO).

### Experimental results

According to the experimental method described above, the inhibitory activity of the compounds disclosed herein against Human M2 and M3 receptors was assayed with AFDX-116 as a positive control compound. The data are summarized in the following table (Table 3).

**Table 3. Inhibitory effect and selectivity of compounds 1-15 prepared in Examples 1-15 on Human M2 and M3**

| Compound | M2 IC₅₀ (nM) | M3 IC₅₀ (nM) | M3/M2 selectivity |
|---|---|---|---|
| AFDX-116 | 165.8 | 2095 | 12 |
| Compound 1 | 3.227 | 610.6 | 189 |
| Compound 2 | 0.733 | 55.92 | 76 |
| Compound 3 | 6.543 | 6816 | 1041 |
| Compound 4 | 1.491 | 132.1 | 88.6 |
| Compound 5 | 2.742 | 673.4 | 245.6 |
| Compound 6 | 0.863 | 723.1 | 838 |
| Compound 7 | 0.714 | 54.15 | 75.87 |
| Compound 8 | 0.896 | 141.2 | 157.7 |
| Compound 9 | 2.981 | 881.4 | 295.7 |
| Compound 10 | 2.581 | 1966 | 762 |
| Compound 11 | 1.857 | 276.6 | 149 |
| Compound 12 | 1.819 | 183.9 | 101 |
| Compound 13 | 5.457 | 260 | 47.6 |
| Compound 14 | 0.8325 | 328.1 | 394.1 |
| Compound 15 | 1.135 | 247 | 217.6 |

As can be seen from Table 3, compounds 1-15 prepared in the examples of the present disclosure all had an excellent M2 inhibitory effect and M3/M2 selectivity.

### Example 17: In Vivo Efficacy Evaluation

Objective: To evaluate the inhibitory effect of compound 2 on the progression of myopia (refractive error) in a guinea pig myopia model. Young guinea pigs have inherent refractive hyperopia, which gradually progresses towards emmetropization during eye development. Form deprivation accelerates the process of emmetropization. In this test, the delaying effect of compound 2 on the process of refractive emmetropization in modeled young guinea pigs was investigated.

Sample preparation: 0.5 wt% compound 2 eye drops were formulated with compound 2 and a 10 wt% aqueous sulfobutylether-beta-cyclodextrin solution.

Grouping: The guinea pigs were evenly divided into 3 groups according to the refractive value of the right eye: a negative control group, a model control group, and a compound 2 group. The guinea pigs in the negative control group did not receive any intervention on the eyes, and the right eyes developed normally.

Modeling: Animals other than the negative control group were modeled. A myopia model was made by suturing the eyelid of the right eye. The modeling procedure is as follows: After hair removal was performed on the right eyes of all animals, the right eyes were sutured in a U-shape to achieve form deprivation and accelerate the process of emmetropization. The guinea pigs were fed for 28 days after the first suture.

Administration: Starting from the day of suture modeling, eye drops were administered to the right eyes of the animals in each group according to settings (the samples for administration are shown in Table 4) at 20 µL /eye once daily for 28 consecutive days.

**Table 4. Administration and dosage design for each group**

| **Group** | **Number of animals (guinea pigs)** | **Sample** | **Dosing volume (µL/eye/time)** | **Frequency of administration** |
|---|---|---|---|---|
| | **Both male and female** | | | |
| Negative control group | 6 | 0.9% sodium chloride injection | 20 | Once daily |
| Model control group | 6 | 0.9% sodium chloride injection | 20 | Once daily |
| Compound 2 group | 6 | 0.5% compound 2 | 20 | Once daily |

Test: Diopter was measured on day 15 and day 29 after modeling using an infrared photorefractor (ST-PR.01, Striatech).

The test results are shown in FIG. 1. Young guinea pigs have inherent refractive hyperopia (positive refractive value), which gradually progresses towards emmetropization, i.e., the process of refractive value going from positive to zero, during eye development (as shown in the negative control group). The modeling (form deprivation) of the present disclosure accelerates the process of emmetropization, and the final refractive value becomes negative (myopia). As can be seen from FIG. 1, the emmetropization progression slope of the model control group was significantly greater than that of the negative control group, demonstrating that the modeling is effective, i.e., the modeling can accelerate emmetropization. The compound 2 group exhibited an increase in the positive refractive value on day 15, indicating significant efficacy; on day 15 of administration, the compound 2 group was superior to the negative control group in slowing down the progression of refraction decrease; and on day 29 of administration, the compound 2 group was comparable to the negative control group in slowing down the progression of refraction decrease. It can be seen that the compound 2 group accelerated the emmetropization process through modeling, but slowed down the emmetropization process after administration, and the emmetropization degree remained comparable to the natural eye development (negative group) 29 days after administration, demonstrating that compound 2 can effectively inhibit the progression of myopia.

In conclusion, the benzodiazepine derivative provided by the present disclosure, as an active component of a pharmaceutical composition, can significantly improve the selectivity for the M2 receptor and significantly reduce the inhibitory effect on the M3 receptor, resulting in a significant increase in the IC₅₀ ratio of M3/M2. In addition, the benzodiazepine derivative has specific pharmacological properties, can effectively prevent or inhibit the progression of myopia without affecting the pupil size, and is beneficial for improving the treatment specificity and patient comfort.

Although the content of the present disclosure has been described in detail with reference to preferred embodiments described above, it should be appreciated that the above description is not to be considered as limiting. Various modifications and substitutions to the present disclosure will become apparent to those skilled in the art after reading the above. The protection scope of the present disclosure is therefore defined by the appended claims.

## Claims

1. An AI-designed benzodiazepine derivative, having a structure of formula I: 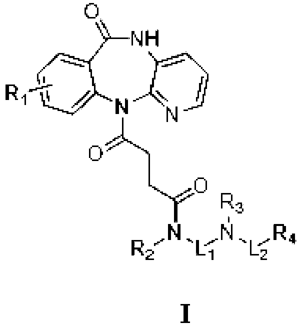 wherein:
R₁ is any one selected from H, deuterium, halogen, and C₁-C₃ alkoxy;
R₂ and R₃ are each independently selected from H, deuterium, and C₁-C₅ alkyl;
R₄ is polycyclic cycloalkyl or polycyclic heterocyclyl;
L₁ is -(CH₂)ₘ-, wherein m is an integer from 1 to 3;
L₂ is a single bond or -(CH₂)ₙ-, wherein n is 1 or 2.

2. The AI-designed benzodiazepine derivative according to claim 1, wherein L₂ is a single bond, and R₃ and R₄, together with the N to which they are linked, may form a C₄-C₂₀ polycyclic heterocyclic structure.

3. The AI-designed benzodiazepine derivative according to claim 1, wherein R₂ and one methylene group in L₁, together with the N to which they are linked, may form a C₄-C₇ heterocyclic structure.

4. The AI-designed benzodiazepine derivative according to any one of claims 1-3, having a structure of formula II: 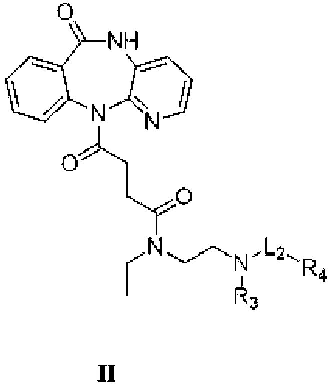 wherein:
R₃ is any one selected from H, deuterium, and C₁-C₅ alkyl;
R₄ is polycyclic cycloalkyl;
L₂ is a single bond or -(CH₂)ₙ-, wherein n is 1 or 2.

5. The AI-designed benzodiazepine derivative according to claim 4, wherein L₂ is a single bond, and R₃ and R₄, together with the N to which they are linked, may form a C₇-C₂₀ heteropolycyclic structure.

6. The AI-designed benzodiazepine derivative according to claim 1, comprising any one of the following:

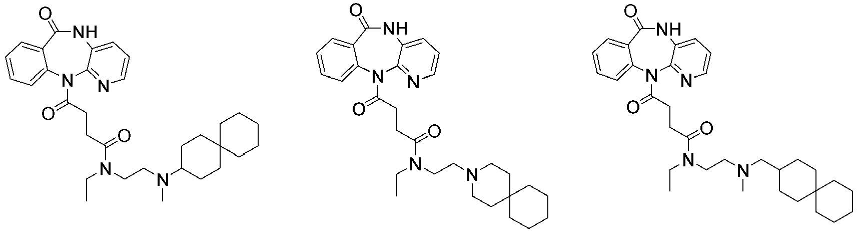

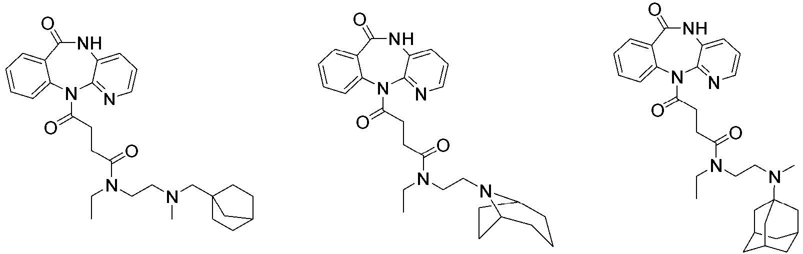

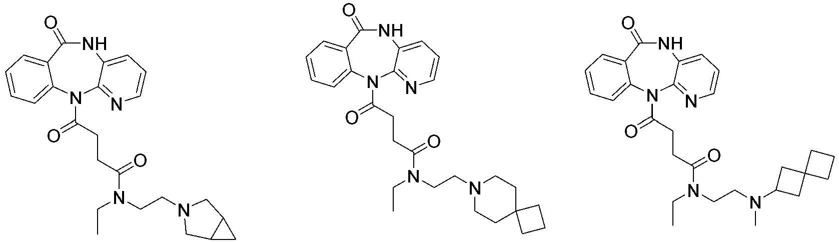

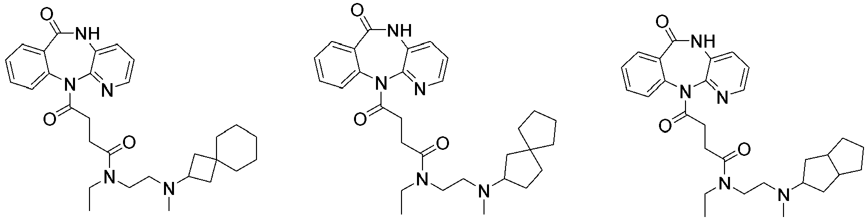

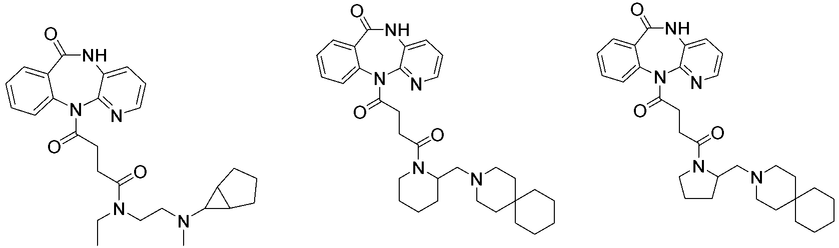

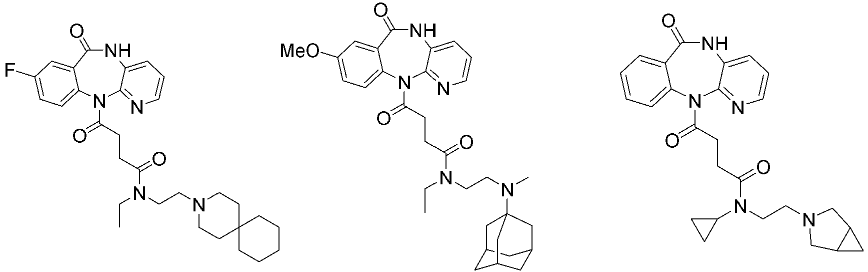

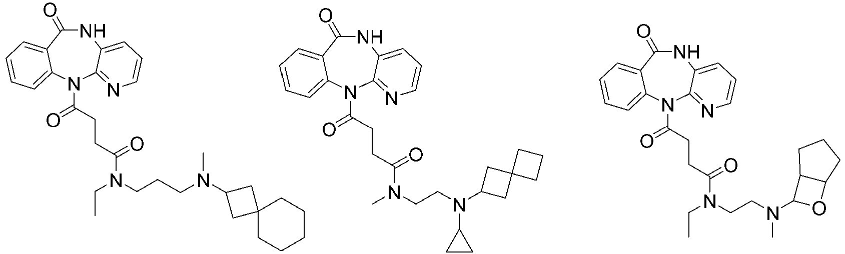

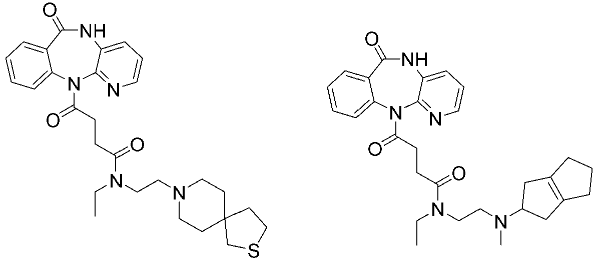

7. A pharmaceutical composition, comprising the AI-designed benzodiazepine derivative according to any one of claims 1-6 as an active ingredient.

8. The pharmaceutical composition according to claim 7, further comprising a pharmaceutical adjuvant.

9. Use of an AI-designed benzodiazepine derivative in the manufacture of a medicament for preventing myopia and/or inhibiting the progression of myopia.

10. The use according to claim 9, wherein the myopia comprises at least one selected from axial myopia, refractive myopia, pseudomyopia, pathological myopia, simple myopia, extreme myopia, severe myopia, high myopia, moderate myopia, low myopia, myopia complicated by glaucoma, myopia at risk of developing glaucoma, or myopia with high intraocular pressure.
